# EUROPEAN PATENT APPLICATION

(11) **EP 2 415 482 A2**
(43) Date of publication of application: **08.02.2012**
(21) Application number: 10758102.7
(22) Date of filing: 30.03.2010
(51) Int. Cl.: A61K 39/395, A61P 35/04

(54) **PHARMACEUTICAL COMPOSITIONS FOR TREATING METASTASIS**

(30) Priority: 02.04.2009 ES 200900907
(71) Applicant: Universidad del Pais Vasco, 48940 Leioa Vizcaya (ES)
(72) Inventor: HERNANDO ECHEVARRÍA, Fernando, E - 48940 Leioa - Vizcaya (ES); RAMÍREZ GARCÍA, Andoni, E - 48940 Leioa - Vizcaya (ES); VIDAL-VANACLOCHA, Fernando, E - 48940 Leioa - Vizcaya (ES); MENDOZA ARTECHE, Lorea, E - 48160 Derio - Vizcaya (ES); GALLOT ESCOBAL, Natalia, E - 48160 Derio - Vizcaya (ES)
(74) Representative: ABG Patentes, S.L.
(86) International application number: PCT/ES2010/070197
(87) International publication number: WO 2010/112657

(57) **Abstract**

The present invention relates to the use of an antifungal agent or of a compound capable of binding to a mannoprotein and blocking the binding of said mannoprotein to an endothelial cell receptor, in the preparation of a pharmaceutical composition for treating a disease associated to an unwanted cell adhesion. Likewise, the present invention also contemplates methods aimed at identifying a compound potentially useful for preventing and/or treating a disease associated to an unwanted cell adhesion, for predicting the probability of a patient suffering from cancer to suffer from metastasis and for designing a personalized therapy for a patient suffering from cancer.

## Description

### Field of the Invention

The present invention relates to the use of an antifungal agent or of a compound capable of binding to a mannoprotein and blocking the binding of said mannoprotein to an endothelial cell receptor in the preparation of a pharmaceutical composition for treating a disease associated to an unwanted cell adhesion. Likewise, the present invention also contemplates methods aimed at identifying a compound potentially useful for preventing and/or treating a disease associated to an unwanted cell adhesion, for predicting the probability of a patient suffering from cancer to suffer from metastasis and for designing a personalized therapy for a patient suffering from cancer.

### Background of the Invention

Glycoproteins are generally called mannoproteins in fungi because the main carbohydrate existing therein is mannose, although other sugar and phosphorus forming phosphodiester bonds can also be found. By paying attention to the differences in the mannoprotein structure two different serotypes can be distinguished: A and B. Mannoproteins are distributed throughout the wall, being accumulated particularly on the surface thereof and in the proximities of the plasma membrane. The mannoproteins play an important role in the adhesion of yeasts to the endothelial cells (Orozco et al., 2000; Infect Immun. 68: 1134-1141).

Some mannoproteins located in the wall of the germ tubes and yeasts are secreted to the medium. The presence of mannoses in the host blood stream is quickly removed due to the presence of mannose receptors in several cell types (Bijsterbosch et al., 1996; Eur J Biochem, 237: 344-349; Stahl & Ezekowitz, 1998; Current Opin Immunol. 10: 50-55; Apostolopoulos & McKenzie, 2001; Curr Mol Med, 1: 469-474). Some of the epitope recognized by the antibodies located in the sugar residues have demonstrated immunosuppressive effects (Nelson et al., 1991; Clin Microbiol Rev, 4: 1-19) and lymphoproliferative effects (Podzorski et al., 1990; J Immunol., 144: 707-716) in addition to having antigenic functions. By means of using specific N-glycosylation inhibitors such as tunicamycin, not only the lack of recognition of these antigenic mannoproteins by the antibodies has been seen, but also the inhibition of the formation of new yolks, the cell division being stopped.

Mannan represents 15-23% of the dry weight of the wall and 40% of the polysaccharides thereof. It is covalently associated to the proteins by means of N-glycosidic and O-glycosidic bonds. This mannoprotein fraction is the main component of the cell wall matrix in which the microfibrillar skeleton of β-glucans and chitin is embedded.

The term mannan has also been used to refer to the main immunodominant soluble component present outside the cell wall called phosphomannoprotein or phosphopeptidemannan complex. This fraction of the cell wall contains D-mannose homopolymers (as the main component), 3-5% of protein and 1-2% of phosphate. By paying attention to the type of binding between the sugar and the protein, the following can be distinguished:
- Highly branched high molecular weight mannose polymers bound to the amino acid asparagine of the peptide by means of a N-glycosidic bond through two N-acetylglucosamines with a highly conserved nucleus both in fungi and in animal cells, and
- Short unbranched mannose oligosaccharides bound to the hydroxyl group of the amino acids serine or threonine of the peptide by means of an O-glycosidic bond.

Metastasis (from Greek: meta, next; and *stasis,* placement) is the cellular mechanism whereby the cells detached from a malignant tumor is transferred from its site of generation to another site without direct anatomic connection where they reimplant. Its production involves a complex process integrated by a succession of interdependent stages (Weiss, L. 1985. Principles of metastasis. New York, N.Y.), wherein the tumor cells manage to colonize other organ territories after being spread through inner cavities or from the lymphatic vascular flow and/or blood stream. Once established as metastasis, they parasite the invaded territory where damaging effects ranging from mechanical to endocrine effects are produced.

The most commonly accepted stages to explain the metastatic process are:
(i) detachment and movement of the tumor cells; depending on the location of the primary tumor, the tumor cells have different capacity to be detached and to leave the tumor source. The detached cells must then reach the blood or lymphatic vessels to be able to be spread towards other distant organs.
(ii)Spreading of the tumor cells; the cells accessing the circulatory stream, in addition to having to adapt to the different blood environmental conditions and to the mechanical effects caused by the circulatory hemodynamics, have to escape from the tumoricidal effect of the immune system.
(iii)Implantation of the metastatic cells in distant organs; In this stage of the metastatic process three consecutive phenomena have been described: the stopping of the circulating tumor cells in the capillary lumen, the extravasation in the surrounding tissue and the proliferation until forming the metastasis.
(iv)Clonogenic proliferation and formation of metastasis; Once extravasated, the proliferation of the metastatic cells is different from that observed in the primary tumor, the tumor cells have a self-regulation conditioned by their own growth factors and their oncogenes.

The existence of receptors for extracellular matrix molecules on the cell surface could be of vital importance to assure the perfect attachment and subsequent proliferation of the tumor cell. The interaction between the circulating tumor cells and the endothelial cells is one of the processes which determines that the metastasis is organ-specific. Adhesion molecules which are expressed both by the tumor cell and by the endothelial cell take part In the process of adhesion between the tumor cell and the vascular endothelium. This fact suggests that the metastatic potential of each tumor cell can be influenced by the type of adhesion molecules which are expressed during the interaction between the tumor cell and the vascular endothelium, therefore the identification of those molecules is considered as one of the fundamental objectives for treating and preventing the tumor spreading.

Although the specific adhesion of the tumor cells to the microvascular endothelium is a verified fact, its implication in the metastasis development is still not known. It has been suggested that the cells adhered to the endothelium could be more protected from the attack of the circulating cytotoxic cells and consequently have higher possibilities of prolonging their half life than staying in the blood stream. The tumor adhesion to the endothelium can also facilitate the cell motility necessary for its extravasation and contribute to another stage of the metastatic process, the tumor invasion of the parenchyma. It has also been proposed that during the metastatic process, the main value of the tumor adhesion to the endothelium corresponds to the translation of signals from the latter to the viable tumor cells, which would serve as stimulus for the intravascular proliferation in the placement sites. In lung, it has been observed that the proliferation of the tumor cells which are adhered to the endothelium causes the formation of intravascular micrometastasis without needing extravasation, which increases the size breaking the vascular wall. Many morphological studies of the tumor adhesion to the endothelium have indicated that the adhesion takes place preferably in the binding area between neighboring endothelial cells. It has been observed that this process continues with an endothelial retraction and the recanalization of the blood vessel around the tumor cells. The endothelial retraction exposes the basal membrane and promotes the tumor adhesion to the extracellular matrix. A simultaneous stimulation of the proteolytic enzyme synthesis and secretion by the endothelial cells could, in turn, favor this process. In this sense, it has been demonstrated that the endothelium damaged by the action of oxygen free radicals, releases active collagenases capable of degenerating the basal membrane and facilitating the tumor invasion. Finally, in relation with the functional implications of the tumor adhesion to the endothelium, the cytotoxic role of the endothelial cells on the tumor cells stands out. This cytotoxicity requires a direct contact between the tumor cells and the endothelium.

The prevention and the therapeutic control of the metastasis is very difficult. The main reason of the medical failure lies on the inexistence of an accurate information on the pathogenesis thereof, which entails a lack of specific resources for the prophylaxis or inhibition thereof.

There is therefore the need of identifying the mechanisms of action and the molecules involved in the metastatic process of the tumor cells which facilitate the prognosis thereof, as well as target therapeutics which facilitate the treatment and prevention thereof.

### Summary of the Invention

In a first aspect, the invention relates to the use of an antifungal agent in the preparation of a pharmaceutical composition for treating a disease associated to an unwanted cell adhesion.

In a second aspect, the invention relates to the use of a compound capable of binding to a mannoprotein, or to an equivalent functional variant thereof, and blocking the binding of said mannoprotein or equivalent functional variant thereof to an endothelial cell receptor in the preparation of a pharmaceutical composition for preventing and/or treating a disease associated to an unwanted cell adhesion.

In a third aspect, the invention relates to a method for identifying a compound potentially useful for preventing and/or treating a disease associated to an unwanted cell adhesion, which comprises contacting an endothelial cell receptor and a mannoprotein or an equivalent functional variant thereof, and selecting a compound blocking the binding of said mannoprotein or equivalent functional variant thereof to said endothelial cell receptor.

In a fourth aspect, the invention relates to the use of a kit comprising a compound capable of binding to a mannoprotein or to an equivalent functional variant thereof, and blocking the binding of said mannoprotein or equivalent functional variant thereof to an endothelial cell receptor for preventing and/or treating a disease associated to an unwanted cell adhesion.

In a fifth aspect, the invention relates to an *in vitro* method for predicting the probability of a patient suffering from cancer to suffer from metastasis, which comprises detecting a yeast or a mannoprotein or an equivalent functional variant thereof in a sample from said patient, wherein if said yeast or mannoprotein or equivalent functional variant thereof is detected, then said patient has a high probability of suffering from metastasis.

In a sixth aspect, the invention relates to an *in vitro* method for designing a personalized therapy for a patient suffering from cancer which comprises detecting a yeast or a mannoprotein or an equivalent functional variant thereof in a sample from said patient, wherein if said yeast or mannoprotein or functional variant thereof is detected, then the patient is a candidate for receiving a treatment based on an antifungal agent or on a compound capable of binding to a mannoprotein or to an equivalent functional variant thereof, and blocking the binding of said mannoprotein or equivalent functional variant thereof to an endothelial cell receptor.

### Brief Description of the Drawings

Figure 1 describes the effect of the morphological state of different strains of *C. albicans* in the relative adhesion of MB16 cells to the HSE (hepatic sinusoidal endothelium): blastoconidia (□) and germ tube (■). (*) Statistically significant differences relative to the control.
Figure 2 describes the effect of the viability of the blastoconidia of *C. albicans* in the relative adhesion of MB16 cells to the HSE. (A) Living blastoconidia, (B) dead blastoconidia due to heat treatment, (C) dead blastoconidia due to treatment with 20 mM metaperiodate and (D) dead blastoconidia due to treatment with 50 mM metaperiodate. (*) Statistically significant differences relative to the control.
Figure 3 describes the results of the flow cytometry where the cellular complexity (SSC) is compared with the size (FSC), and the fluorescence (FL1) is compared with the cell size (FSC). R1: living population. R2: dead with 20 mM metaperiodate. R3: dead with 50 mM metaperiodate.
Figure 4 shows the carbohydrate/protein ratio in blastoconidia and germ tubes of different strains of *C. albicans*. Blastoconidia (□) and germ tube (■). (*) Statistically significant differences.
Figure 5 describes the effect of different concentrations of living and dead *C. albicans* yeasts by means of oxidizing with 20 and 50 mM metaperiodate in the production of IL-18 by the HSE. (*) Significant differences relative to the control.
Figure 6 shows the photographs of the HSE cells after being incubated with commercial mannan (A), crude extract (B), protein fraction (C) and mannoprotein fraction (D).
Figure 7 describes the relative adhesion of MB16 cells to the HSE incubated with commercial mannan and the purified mannoprotein fraction of *C. albicans* (MNF). (*) Significant differences with respect to the control.
Figure 8 describes the relative adhesion of MB16 cells to the HSE incubated with the protein fraction, the crude extract and the mannoprotein fraction (□). Release of TNF-α incubated with the protein fraction, the crude extract and the mannoprotein fraction (■). (*) Significant differences with relative to the control.
Figure 9 shows the increase of the tumor adhesion induced by the incubation of the mouse HSE with *C. albicans* or the mannoproteins thereof in basal medium and by using an antibody blocking the mannose receptor at two different concentrations. The adhesion is expressed in relative units. (*) Significant differences relative to the control.
Figure 10 shows the increase of the production of IL-18 induced by the incubation of the HSE with C. *albicans* or the mannoproteins thereof in basal medium and by using an antibody blocking the mannose receptor. (*) Significant differences with respect to the control. (**) Significant reduction relative to the situation without blocking agent.
Figure 11 shows a two-dimensional electrophoresis of the crude extract of *C. albicans* viewed by means of silver staining.
Figure 12 shows a two-dimensional electrophoresis of the protein fraction of *C. albicans* viewed by means of silver staining.
Figure 13 shows a two-dimensional electrophoresis of the mannoprotein fraction of *C. albicans* viewed by means of silver staining.
Figure 14 describes a two-dimensional Western blot of the crude extract of *C. albicans* developed by means of chemiluminescence using Con A-HRP.
Figure 15 shows a 1D electrophoresis of the 5 mannoprotein subfractions obtained by means of preparative electrophoresis from the crude extract stained with silver (A). Detection of the 5 subfractions with Con A-HRP and developed with chemiluminescence after performing the affinity chromatography from those obtained by means of preparative electrophoresis (B). F1: 0 - 30 kDa, F2: 30 - 45 kDa, F3: 45 - 66 kDa, F4: 66 - 115 kDa, F5: >115 kDa.
Figure 16 describes the carbohydrate/protein proportion of each of the mannoprotein subfractions obtained. MNF1: 0 - 30 kDa, MNF2: 30 - 45 kDa, MNF3: 45 - 66 kDa, MNF4: 66 - 115 kDa, MNF5: >115 kDa.
Figure 17 shows the relative adhesion of B16 melanoma cells to the HSE incubated with the subfractions separated according to their molecular weight. MNF1: 0-30 kDa, MNF2: 30 - 45 kDa, MNF3: 45 - 66 kDa, MNF4: 66 - 115 kDa, MNF5: >115 kDa. (*) Significant differences with respect to the increase induced by the commercial mannan.
Figure 18 shows a 2D electrophoresis of the MNF stained with coomassie blue with the numbered sequenced points.
Figure 19 shows a 1D electrophoresis of the subfractions obtained by means of preparative isoelectric focusing from the crude extract stained with silver (A). Detection of the subfractions with Con A-HRP and developed with chemiluminescence after performing the affinity chromatography (B). F1: <4.25, F2: 4.25 - 5, F3: 5 - 5.6, F4: >5.6.
Figure 20 shows the relative adhesion of B16 melanoma cells to the HSE incubated with the mannoprotein subfractions separated according to their isoelectric point. MNF1: <4.25, MNF2: 4.25 - 5, MNF3: 5 - 5.6, MNF4: >5.6. (*) Significant differences relative to the commercial mannan.
Figure 21 shows a visual reproduction of the fractioning of the mannoproteins of *C. albicans* made according to the molecular weight and the isoelectric point.
Figure 22 shows a two-dimensional electrophoresis of the mannoproteins from the strains of *C. albicans* UPV1413 (top), NCPF3153 (center) and UPV1360 (bottom) stained by means of coomassie blue. The protein disulfide isomerase (A), the basic subunit of the enolase (B) and the aminopeptidase (C) are labeled.
Figure 23 shows the proliferation of tumor cells in control medium supplemented with fetal bovine serum (FBS) and with the mannoprotein fraction (MNF).
Figure 24 shows the proliferation of tumor cells after 48 hours of incubation in control medium supplemented with fetal bovine serum(FBS) and with the mannoprotein fraction MNF3 (45-66 kDa).
Figure 25 shows the relative adhesion of B16 melanoma cells to the HSE treated with the MNF blocking and not blocking the HSE IL-1β receptor with IL-Ra (□). Release of TNF-α by the HSE treated with the MNF blocking and not blocking the HSE IL-1β receptor with IL-Ra (■).
Figure 26 shows a two-dimensional Western blot of the mannoproteins of *C. albicans* UPV1413 (A) evaluated in comparison to the anti-mouse IL-1β neutralizing antibody developed by means of chemiluminescence.

### Detailed Description of the Invention

The inventors of the present invention have discovered that the inflammation caused in response to the binding of *Candida albicans* to the endothelial receptors does not only have the effect of attracting phagocytes to the infected area to remove the invader, but surprisingly directly increases the risk of adhesion of the tumor cells to that same area in patients suffering from cancer, which involves the start of a metastatic process. Likewise, the inventors have discovered that said capacity of *C. albicans* to directly increase the risk of suffering from metastasis is due to the mannoproteins present in the mannoprotein fraction of the yeast, which interact with the endothelial cell receptors, such as the proinflammatory cytokine receptors, such as for example, the IL-1β receptor, causing not only the inflammatory response to the infection, but also the adhesion of the tumor cells to the endothelium starting the metastatic process of the tumor.

Therefore, in one aspect, the present invention relates to the use of an antifungal agent in the preparation of a pharmaceutical composition for treating a disease associated to an unwanted cell adhesion.

In the context of the present invention, a "disease associated to an unwanted cell adhesion" includes the progress and the metastasis of cancer and tumors. Non-limiting illustrative examples of diseases associated to an unwanted cell adhesion and which can be treated according to the teachings of the present invention include cancer, metastasis, restenosis, diseases causing a cellular immune response and inflammatory diseases.

Therefore, in a particular embodiment, the disease associated to an unwanted cell adhesion is a tumor process, a metastatic process, an inflammatory process, restenosis or a disease causing a cellular immune response.

In the context of the present invention, "tumor process" is understood as those diseases characterized by the development of a tumor, such as the case of cancer. Examples of tumors which can be treated with the pharmaceutical composition described in the present invention include, without being limiting, lung tumor, prostate tumor, head and neck tumor, testicle tumor, brain tumor, skin tumor, colon tumor, rectum tumor, esophagus tumor, tracheal tumor, pancreatic tumor, liver tumor, breast tumor, ovary tumor, tumor of the lymphatic system, leukemia, cervical tumor, melanoma, kidney tumor, bladder tumor, thyroid tumor, bone tumor and stomach tumor.

In the context of the present invention, the term "metastatic process" and "metastasis" are considered the same and are used indistinctly. Metastasis is understood as the process whereby cancerous cells propagate to other organs different from where the primary tumor was formed by means of lymphatic channels and blood vessels. The person skilled in the art will realize that the pharmaceutical composition of the invention can be used in the inhibition of any type of metastasis formed from virtually any type of tumor.

The invention contemplates the use of antifungal agents in the preparation of a pharmaceutical composition for preventing or treating the metastasis in any organ of the body, such as head and neck (face, eye, mouth, tongue, teeth, nose, ears, scalp, larynx, pharynx, salivary glands, meninges, brain, thyroid gland and parathyroid), shoulder and backbone (vertebrae and spinal cord), thorax (mammary glands, ribs, lungs, heart, mediastinum, esophagus and diaphagram), abdomen (peritoneum, stomach, duodenum, intestine, colon, liver, kidney, adrenal gland, appendix and pancreas), pelvis (sacrum, coccyx, ovaries, Fallopian tube, uterus, vagina, vulva, clitoris, perineum, urinary bladder, testicles, rectum and penis) and extremities(muscle, bone, nerves, hand, wrist, elbow, shoulder, hip, knee or ankle).

On the other hand, the tumors from which the metastasis is originated include, without being limiting, lung tumor, prostate tumor, head and neck tumor, testicle tumor, brain tumor, skin tumor, colon tumor, rectum tumor, esophagus tumor, tracheal tumor, pancreatic tumor, liver tumor, breast tumor, ovary tumor, tumor of the lymphatic system, leukemia, cervical tumor, melanoma, kidney tumor, bladder tumor, thyroidal tumor, bone tumor and stomach tumor. In a form of preferred embodiment, the metastatic process is originated from a liver tumor.

In the context of the present invention, "an inflammatory process" or "inflammatory disease" is understood as that disease causing inflammation as a consequence of a cell proliferation associated with a proliferative dysfunction, e.g., proliferative glomerulonephritis , lupus erythematosus, scleroderma, temporal arthritis, thromboangiitis, mucocutaneous lymph node syndrome, etc. In a particular embodiment, the inflammatory disease or inflammatory process is a cytokine mediated inflammatory process.

In the context of the present invention, "restenosis" is understood as that disease including an excessive proliferation and migration of cells as a result of the release of growth factors produced by a mechanical damage of the endothelial cells forming the coronary arteries.

In the context of the present invention, "a disease causing a cellular immune response" is understood as that disease causing a T-lymphocyte mediated adaptive immunologic response which acts like a defense mechanisms against intracellular microorganisms, e.g., virus, some bacteria and fungi, capable of surviving and proliferating inside the phagocytes and other host cells, site which cannot be accessed by the circulating antibodies, etc. The defense against this type of infections depends on the cellular immunity inducing the destruction of the resident microorganism in the phagocytes or of the infected cells.

The antifungal or antimycotic agent used in the preparation of the pharmaceutical composition of the present invention encompasses any substance capable of producing an alteration such of the fungal cell structures that it attains an inhibition in their development, altering the viability or survival capacity either directly or indirectly thereof.

The antifungal or antimycotic agents include a wide variety of substances with different chemical structures and mechanisms of action. The classification is made according to conventional criteria paying attention to their structure, their origin (if they are produced by living organisms or derived from chemical synthesis), their spectrum of action (wide or restricted) or their site of action.

Examples of antifungal agents include, without being limiting, (i) polyenes, such as nystatin, natamycin and amphotericin B, (ii) azoles, such as imidazoles (miconazole, clotrimazole), triazoles (fluconazole, itraconazole, ketoconazole) and second generation triazoles (voriconazole, ravuconazole, posaconazole), (iii) allylamines, such as terbinafine and naftifine, (iv) lipopeptides, such as papulacandins, glycosylated triterpenes and echinocandins (caspofungin, anidulafungin, micafungin) and (v) fluorinated pyrimidines, such as flucyitosine. Other antifungal agents include, for example, potassium iodide, cyclopirox, tolnaftate and griseofulvin. In a particular embodiment, the antifungal agent is selected from a polyene, an azole, an allylamine, a lipopeptide and a fluorinated pyrimidine.

In another aspect, the invention relates to the use of a compound capable of binding to a mannoprotein, or to an equivalent functional variant thereof, and blocking the binding of said mannoprotein or equivalent functional variant thereof to an endothelial cell receptor in the preparation of a pharmaceutical composition for preventing and/or treating a disease associated to a cell adhesion.

In the present invention, "mannoprotein" is understood as a glycoprotein wherein the main carbohydrate existing therein is mannose. The mannoproteins are mainly found forming part of the fungi and yeast cell wall where they play an important role in the adhesion of the yeasts to the endothelial cells and endothelia. Thus, if a crude extract of a yeast such as C. *albicans* is separated by means of, for example, an affinity chromatography, two fractions can be obtained: a mannoprotein fraction (MNF) and a protein fraction (PF). As understood by the person skilled in the art, PF is that which does not contain or contains a small amount of mannoproteins.

Thus, in a particular embodiment, the mannoprotein comes from the mannoprotein fraction of a yeast.

Most of the yeasts are opportunistic pathogens which may cause infections compromising the immune system of affected people. Examples of yeasts include, without being limiting, yeasts from the genus *Cryptococcus* such as *C*. *neoformans,* or yeasts from the genus *Candida,* such as *C*. *albicans, C*. *tropicalis, C*. *stellatoidea, C. glabrata, C*. *krusei, C*. *parapsilosis, C. guilliermondii, C. viswanathii, C. lusitaniae Candida famata, C. holmii, C. kefyr, C. pelliculosa, C. rugosa, C. utilis, C. zeylanoides, Rhodotorula mucilaginosa, R. minuta, R. glutinis, R. mucilaginosa* and *Trichosporon cutaneum,* the yeast *Saccharomyces cerevisiae,* etc.

In another particular embodiment, the mannoprotein comes from the mannoprotein fraction of a yeast, preferably of a yeast from the genus *Candida,* more preferably, *C. albicans.*

As is known by the person skilled in the art, the MNF of a yeast (or other organism) can be obtained by any of the methods described in the state of the art for separating proteins such as, without being limiting, chromatography (for example, ion exchange chromatography, affinity chromatography (in Con-A Sepharose column activated with cyanogen bromide), hydrophobic interaction chromatography, gel filtration chromatography, HPLC), electrophoretic methods (preparative isoelectric focusing, preparative electrophoresis in polyacrylamide-SDS gels (one- and two-dimensional), preparative isoelectric focusing in the Rotofor), differential solubility (precipitation with ammonium sulfate), preparative ultracentrifugation in sucrose gradient. If it is necessary to concentrate the protein or to remove salts and ions which may be harmful, known techniques such as lyophilization or ultrafiltration are resorted to.

In the present invention, the mannoproteins are separated by one-dimensional electrophoresis which allows separating them according to their molecular weight (Mw). Thus, by using the techniques mentioned herein, the analysis of the composition of the C. *albicans* MNF has given rise to 5 subfractions of different molecular weight, MNF1 (0 - 30 kDa), MNF2 (30-45 kDa), MNF3 (45-66 kDa), MNF4 (66-115 kDa) and MNF5 (>115 kDa). In a particular embodiment, the mannoprotein fraction comprises at least one mannoprotein having a molecular weight between 30 and 45 kDa or between 45 and 66 kDa.

Thus, in a particular embodiment, the mannoprotein having a molecular weight between 45 and 66 kDa is a mannoprotein selected from disulfide isomerase, zinc protein, aminopeptidase Y, mitochondrial ketol acid-reductoisomerase, Tu elongation factor, Tu translation elongation factor, actin, enolase, alcohol dehydrogenase, 6-phosphogluconate dehydrogenase, fructose bisphosphate aldolase, 3-phosphoglycerate kinase, isocytrate dehydrogenase, GDP-mannose pyrophosphorylase, glutathione dependent formaldehyde dehydrogenase and ubiquinol cyt-c reductase.

Once the MNF is separated into the corresponding subfractions, the mannoproteins comprising a subfraction can be identified, for example, by means of a two-dimensional electrophoresis which allows separating the proteins not only based on their molecular weight but also according to their isoelectric point (pI), to obtain a peptide fingerprint.

Therefore, in a particular embodiment, the mannoprotein fraction comprises at least one mannoprotein having a molecular weight between 45 and 66 kDa and an isoelectric point between 5 and 5.6.

In another yet more particular embodiment, the mannoprotein having a molecular weight between 45 and 66 kDa and an isoelectric point between 5 and 5.6 comprises at least one mannoprotein selected from aminopeptidase Y, mitochondrial ketol acid-reductoisomerase, Tu elongation factor, Tu translation elongation factor, actin, enolase, ubiquinol cyt-c reductase and alcohol dehydrogenase.

The present invention is based on the discovery of the capacity of C. *albicans* or of its mannoproteins for inducing the adhesion of tumor cells to endothelial cells, thus promoting the metastasis of the tumor. Therefore, the present invention relates to the use of a compound capable of binding to a mannoprotein and of blocking the binding of said mannoprotein to an endothelial cell receptor in the preparation of a pharmaceutical composition for preventing and/or treating a disease associated to an unwanted cell adhesion.

In the context of the present invention, "a compound capable of binding to a mannoprotein and of blocking the binding of said mannoprotein to an endothelial cell receptor" is understood as that compound which specifically recognizes a mannoprotein, or an equivalent functional variant thereof, and is bound covalently thereto thus blocking the binding of said mannoprotein or equivalent functional variant thereof to an endothelial cell receptor, thus inhibiting the cell adhesion of the tumor cells to the endothelial cells.

Examples of compounds capable of specifically recognizing a mannoprotein and covalently binding thereto are, for example, the antibodies such as those described in the patent application WO03/089607. Therefore, in a particular embodiment, the compound capable of binding to a mannoprotein or to an equivalent functional variant thereof, and blocking its binding to a receptor of the endothelia is an antibody.

The term "antibody" must be interpreted in a broad sense and includes multispecific antibodies, polyclonal antibodies, monoclonal antibodies and Fab fragments thereof, F(ab' )₂, provided that they can bind specifically to a mannoprotein or to an equivalent functional variant thereof, and blocking the binding of said mannoprotein or equivalent functional variant thereof to an endothelial cell receptor, thus inhibiting the cell adhesion of the tumor cells to the endothelial cells. Examples of antibodies which can be used in this context are, for example and not limited to, polyclonal antibodies, monoclonal antibodies, recombinant antibodies, chimeric antibodies, humanized antibodies, completely human antibodies, etc.

The polyclonal antibodies are originally heterogeneous antibody molecule mixtures produced in the serum of animals which have been immunized with an antigen. They also include monospecific polyclonal antibodies obtained from heterogeneous mixtures, for example, by means of column chromatography with peptides of an individual epitope of the antigen of interest.

A monoclonal antibody is a homogenous population of specific antibodies for an individual epitope of the antigen. These monoclonal antibodies can be prepared by means of conventional techniques which have been described, for example, in Köhler and Milstein [Nature, 1975; 256: 495-497] or Harlow and Lane ["Using Antibodies. A Laboratory Manual" by E. Harlow and D. Lane, Editor: Cold Spring Harbor Laboratory Press, Cold Spring Harbor, New York; 1998 (ISBN 978-0879695439)].

A chimeric antibody is a monoclonal antibody constructed by means of cloning or recombining antibodies of different animal species. In a typical but non-limiting configuration of the invention, the chimeric antibody includes a part of a monoclonal antibody, generally the variable (Fv) region which includes the antigen recognition and binding sites and the other part corresponding to a human antibody, generally the part which includes the constant region and the region adjacent to the constant region.

A completely human antibody is an antibody or antibodies which have been produced in transgenic animals with a human immune system or by means of *in vitro* immunization of human immune cells (including both genetic immunization and traditional immunization with or without adjuvants and pure or impure antigen; or by means of any method to expose the antigen to the immune system) or by means of native/synthetic libraries produced from human immune cells. These antibodies can be obtained and screened from transgenic animals (mice, for example) in which genes of the human immunoglobulins have been cloned and are immunized with the target antigen (the mannoprotein or an equivalent functional variant thereof). These antibodies can be obtained by means of screening human single-chain variable regions (scFv) or antigen binding regions (Fab) presented in phages and subsequently cloning and injecting into a human antibody or by means of any other method known by the person skilled in the art, to produce and present the libraries generated by means of cloning the variables regions of both chains and subsequently cloning/mutating the same to generate antibody libraries.

A humanized antibody is a monoclonal antibody constructed by means of cloning and injecting the hypervariable complementarity determining regions (CDR) of a murine monoclonal antibody in a human antibody substituting his own hypervariable CDR regions.

The antibody with capacity of binding specifically to a mannoprotein can be obtained by means of conventional recombinant or genetic engineering techniques, conventional antibody production techniques, extraction and purification of biological fluids or tissues, or any other conventional technique to obtain proteins and antibodies which are widely known by the persons skilled in the art. Said techniques include, without this being any limitation: animal immunization techniques including transgenic animals for the human immunoglobulin genes, production of monoclonal antibodies by means of hybridomas, production by means of antibody libraries which can be native libraries, synthetic libraries, or derived from organisms immunized against the antigen of interest and which could be screened by means of different presentation methods (presentation in phage, presentation in ribosome, etc.) and subsequently, by means of genetic engineering techniques, they could be redesigned and expressed in vectors designed to produce recombinant antibodies with different sizes, composition and structure. A review of the main methods to produce and purify antibodies can be found, for example, in:
- "Handbook of Therapeutic Antibodies", by S. Dübel. Editor: Wiley-VCH, 2007, Vol: I to III (ISBN 978-3527314539);
- "Antibodies: Volume 1: Production and Purification" by G. Subramanian Ed., Editor: Springer, 1a Ed, 2004 (ISBN 978-0306482458);
- "Antibodies: Volume 2: Novel Technologies and Therapeutic Use" by G. Subramanian Ed., Editor: Springer, first edition, 2004 (ISBN 978-0306483158);
- "Molecular Cloning: a Laboratory Manual", of J. Sambrook and D.W. Russel Eds., Editor: Cold Spring Harbor Laboratory Press, third edition, 2001 (ISBN 978-0879695774).

In the present invention, "equivalent functional variant of a mannoprotein" is understood as a protein the amino acid sequence of which (i) is substantially homologous to the amino acid sequence of said mannoprotein and (ii) performs the same functions as said mannoprotein. The functional similarity of one protein with another specific protein can be determined by means of interference tests with the expression of the gene encoding the specific protein which, upon reducing the expression, would reduce the activity of that protein, and the subsequent recovery of the activity by means of expressing the sequence of the other protein. These experiments are performed using interference RNA sequences specific for and complementary to the sequence of the specific protein and expression vectors incorporating the specific sequence of the other protein regulated by an inducible or non-inducible promoter.

An assay to ascertain whether a variant of a mannoprotein such as has been defined herein is functionally equivalent to a mannoprotein, and therefore, performs the same function (promotes the cell adhesion of the tumor cells to the epithelial cells), is a tumor adhesion assay, in this case induced by the variant of a mannoprotein to be assayed to ascertain whether it is functionally equivalent to the mannoprotein of which it is a variant, wherein the adhesion is measured using a method based on the fluorescence measurement. Briefly, said assay comprises labeling tumor cells, for example, MB16 melanoma cells with a fluorescent agent (for example, BCECF-AM) and subsequently, contacting endothelial cells incubated with the variant to be assayed with the labeled tumor cells. Once the adhesion time ended, the co-cultures were excited with light suitable for the marker used (in case of using BCECF-AM the wavelength to be applied is 488 nm) and the fluorescence emitted by the marker incorporated to the tumor cells (for example, by means of silver barrier) is recorded. After several washings to remove the endothelial cells not adhered to the endothelial cells, adhesion medium is added and the fluorescence emitted is read again. Thus, the tumor relative adhesion can be measured which, compared with the control, allows identifying a variant as functionally equivalent if the relative data of fluorescence are greater than the data obtained by the control. More details on the assay can be found in the material and methods section of the examples accompanying the present description.

An amino acid sequence is substantially homologous to a particular amino acid sequence when it has a degree of identity of at least 70%, advantageously of at least 75%, typically of at least 80%, preferably of at least 85%, more preferably of at least 90%, yet more preferably of at least 95%, 97%, 98% or 99%, with respect to said particular amino acid sequence. The degree of identity between two amino acid sequences can be determined by conventional methods, for example, by means of standard sequence alignment algorithms known in the state of the art, such as, for example BLAST [Altschul S.F. et al. Basic local alignment search tool. J Mol Biol. 1990 Oct 5; 215(3):403-10].

The person skilled in the art understands that, the mutations in the nucleotide sequence of a protein which give rise to conservative amino acid substitutions in positions noncritical for the functionality of the protein, are evolutionarily neutral mutations which do not affect the overall structure or the functionality thereof. Said variants fall within of the scope of the present invention. Those variants having insertions, deletions or modifications of one or more amino acids with respect to a mannoprotein and conserve, furthermore, the same functions as said mannoprotein are also included within of the scope of the invention.

Therefore, as it is used herein, the term "equivalent functional variant" also includes any fragment of a mannoprotein. The term "fragment" relates to a peptide comprising a portion of a protein. In this case, a functionally equivalent fragment of a mannoprotein is a peptide or protein comprising a portion of a mannoprotein and the same functions as said mannoprotein. An assay to ascertain whether a fragment of a mannoprotein is functionally equivalent to said mannoprotein has been explained above in the present description.

As has been previously explained, the capacity of *C*. *albicans* to directly increase the risk of a patient suffering from cancer to suffer from metastasis is due to the fact that the mannoproteins present in the mannoprotein fraction of the yeast interact with the endothelial cell receptors, causing an increase of the adhesion of the tumor cells to the endothelial cells.

In the context of the present invention, "endothelial cell receptor" is understood as those molecule receptors which are found on the surface of the endothelial cells and which allows the binding of ligands to the cells, thus triggering signal cascades activating or deactivating different cellular processes such as cell proliferation, apoptosis, cell aggregation, proinflammatory response, etc....

In a particular embodiment, the endothelial cell receptor is a proinflammatory cytokine receptor. Examples of proinflammatory cytokine receptors include, without being limiting, the receptor of the TNF-α, the IL-1 receptor, the IL-1β receptor, the IL-6 receptor, the IL-8 receptor, etc. In a still more particular embodiment, the proinflammatory cytokine receptor is the interleukin 1-beta receptor (IL1- β).

As has been indicated above, the invention relates to the use of a compound capable of binding to a mannoprotein and blocking the binding of said mannoprotein to an endothelial cell receptor in the preparation of a pharmaceutical composition for preventing and/or treating a disease associated to an unwanted cell adhesion or a disease caused by an infectious agent.

In a particular embodiment, the disease associated to an unwanted cell adhesion is a tumor process, a metastatic process, an inflammatory process preferably mediated by cytokines, restenosis or a disease causing a cellular immune response.

In another particular embodiment, the metastatic process is originated from liver tumor.

The meaning of said terms as well as the examples thereof has been discussed above.

In a particular embodiment, the pharmaceutical composition comprises one or more pharmaceutically acceptable excipients and, additionally, in another particular embodiment, it may comprise an antifungal compound such as that mentioned above in the present specification.

For use in medicine, the pharmaceutical composition of the invention can be formulated in the form of prodrug, salt, solvate or clathrate, either in an isolated form or in combination with additional active ingredients. The excipients preferred for use in the present invention include sugar, starches, celluloses, rubbers and proteins. The pharmaceutical composition can be formulated in a solid pharmaceutical dosage form (for example, tablets, capsules, sugar coated tablets, granules, suppositories, amorphous or crystalline sterile solids which can be reconstructed to provide liquid forms, etc.), liquid (for example, solutions, suspensions, emulsions, elixirs, lotions, unguent etc.) or semisolid (gels, ointments, creams and the like). The examples of pharmaceutically acceptable supports are known in the art and they include saline solutions buffered with phosphate, water, emulsions such as oil in water emulsions, different types of wetting agents, sterile solutions, etc. The compositions comprising said supports can be formulated by means of conventional processes known in the state of the art.

In another aspect, the invention relates to the use of a kit comprising a compound capable of binding to a mannoprotein, or to an equivalent functional variant thereof, and blocking the binding of said mannoprotein, or equivalent functional variant thereof, to an endothelial cell receptor for preventing and/or treating a disease associated to an unwanted cell adhesion.

In the present invention, a "kit" is understood as a product containing the different active ingredients or compounds of the invention (i.e., a compound capable of binding to a mannoprotein, or to an equivalent functional variant thereof, and blocking the binding of said mannoprotein or equivalent functional variant thereof to an endothelial cell receptor) forming the composition packaged such that it allows its transportation, storage and the simultaneous and sequential administration thereof. Thus, the kits of the invention can contain one or more suspensions, tablets, capsules, inhalers, syringes, patches and the like, which contain the active ingredients of the invention and which can be prepared in a single dose or as multiple doses. The kit can additionally contain a support suitable for resuspending the compositions of the invention such as an aqueous medium, such as saline solution, Ringer's solution, Ringer's lactate solution, dextrose and sodium chloride, water soluble media such as alcohol, polyethylene glycol, propylene glycol and water insoluble supports such as corn oil, cottonseed oil, peanut oil, sesame oil, ethyl oleate, isopropyl myristate and benzyl benzoate. Other components which can be present in the kit is a container which allows maintaining the formulations of the invention within determined limits. The materials suitable for preparing such containers include glass, plastic, polyethylene, polypropylene, polycarbonate and the like, bottles, vials, paper, bags and the like.

The kit of the invention can additionally contain instructions for the simultaneous, sequential or separated administration of the different pharmaceutical formulations present in the kit. Therefore, in a form of particular embodiment, the kit of the invention further comprises instructions for the simultaneous, sequential or separated administration of the different components. Said instructions can be found in the form of printed material or in the form of electronic support which can store the instructions such that they can be read by a subject, such as electronic storage means (magnetic discs, tapes and the like), optical means (CD-ROM, DVD) and the like. The means can additionally or optionally contain Internet websites providing said instructions.

All the particular embodiments defining this inventive aspect have been discussed, described and explained in previous inventive aspects and therefore more commentaries with regards to this is not necessary.

Therefore, in a particular embodiment of the kit of the invention, the mannoprotein is from the mannoprotein fraction of a yeast, preferably, of a yeast from the genus candida, more preferably from *Candida albicans.*

In another particular embodiment, the mannoprotein fraction comprises at least one mannoprotein having a molecular weight between 30 and 45 kDa or between 45 and 66 kDa.

In another particular embodiment, the mannoprotein having a molecular weight between 45 and 66 kDa of molecular weight is a mannoprotein selected from disulfide isomerase, zinc protein, aminopeptidase Y, mitochondrial ketol acid-reductoisomerase, Tu elongation factor, Tu translation elongation factor, actin, enolase, alcohol dehydrogenase, 6-phosphogluconate dehydrogenase, fructose bisphosphate aldolase, 3-phosphoglycerate kinase, isocytrate dehydrogenase, GDP-mannose pyrophosphorylase, glutathione dependent formaldehyde dehydrogenase and ubiquinol cyt-c reductase.

In another particular embodiment, the mannoprotein fraction comprises at least one mannoprotein having a molecular weight between 45 and 66 kDa and an isoelectric point between 5 and 5.6.

In another particular embodiment, the mannoprotein having a molecular weight between 45 and 66 kDa and an isoelectric point between 5 and 5.6 comprises at least one mannoprotein selected from aminopeptidase Y, mitochondrial ketol acid-reductoisomerase, Tu elongation factor, Tu translation elongation factor, actin, enolase, ubiquinol cyt-c reductase and alcohol dehydrogenase.

In another particular embodiment, the endothelial cell receptor is a proinflammatory cytokine receptor, preferably the interleukin 1-beta receptor (IL1-(β).

In another particular embodiment, the disease associated to an unwanted cell adhesion is a tumor process, a metastatic process, an inflammatory process preferably mediated by cytokines, restenosis or a disease causing a cellular immune response.

In another particular embodiment, the metastatic process is originated from liver tumor.

In another particular embodiment, the compound capable of binding to a mannoprotein, or an equivalent functional variant thereof, and blocking its binding to a proinflammatory cytokine receptor is an antibody.

The discovery made by the inventors of the present invention based on the capacity of C. *albicans* or of the mannoproteins obtained from said yeast for inducing the adhesion of tumor cells to the endothelial cells through the interaction with the endothelial cell receptors, and thus promoting the metastasis, can be also used to identify a compound useful in the prevention and/or the treatment of a disease associated to an unwanted cell adhesion, since any compound blocking said interaction will prevent the adhesion and therefore the metastasis.

Thus, in another aspect, the invention relates to a method for identifying a compound potentially useful for preventing and/or treating a disease associated to an unwanted cell adhesion, which comprises contacting an endothelial cell receptor and a mannoprotein or an equivalent functional variant thereof, and selecting a compound blocking the binding of said mannoprotein or equivalent functional variant thereof to said endothelial cell receptor.

Such as has been expressed above, all the particular embodiments defining this inventive aspect have been discussed, described and explained in previous inventive aspects and therefore more commentaries with regards to this is not necessary.

Therefore, in a particular embodiment, the mannoprotein is from the mannoprotein fraction of a yeast, preferably of a yeast from the genus candida, more preferably from *Candida albicans.*

In another particular embodiment, the mannoprotein fraction comprises at least one mannoprotein having a molecular weight between 30 and 45 kDa or between 45 and 66 kDa.

In another particular embodiment, the mannoprotein having a molecular weight between 45 and 66 kDa of molecular weight is a mannoprotein selected from disulfide isomerase, zinc protein, aminopeptidase Y, mitochondrial ketol acid-reductoisomerase, Tu elongation factor, Tu translation elongation factor, actin, enolase, alcohol dehydrogenase, 6-phosphogluconate dehydrogenase, fructose bisphosphate aldolase, 3-phosphoglycerate kinase, isocytrate dehydrogenase, GDP-mannose pyrophosphorylase, glutathione dependent formaldehyde dehydrogenase and ubiquinol cyt-c reductase.

In another particular embodiment, the mannoprotein fraction comprises at least one mannoprotein having a molecular weight between 45 and 66 kDa and an isoelectric point between 5 and 5.6.

In another particular embodiment, the mannoprotein having a molecular weight between 45 and 66 kDa and a isoelectric point between 5 and 5.6 comprises at least one mannoprotein selected from aminopeptidase Y, mitochondrial ketol acid-reductoisomerase, Tu elongation factor, Tu translation elongation factor, actin, enolase, ubiquinol cyt-c reductase and alcohol dehydrogenase.

In another particular embodiment, the endothelial cell receptor is a proinflammatory cytokine receptor, preferably the interleukin 1-beta receptor (IL1-(β).

In another particular embodiment, the disease associated to an unwanted cell adhesion is a tumor process, a metastatic process, an inflammatory process preferably mediated by cytokines, restenosis or a disease causing a cellular immune response.

In another particular embodiment, the metastatic process is originated from liver tumor.

In another particular embodiment, the compound capable of binding to a mannoprotein, or an equivalent functional variant thereof, and blocking its binding to a proinflammatory cytokine receptor is an antibody.

In another aspect, the invention relates to an in *vitro* method for predicting the probability of a patient suffering from cancer to suffer from metastasis, which comprises detecting a yeast or a mannoprotein or an equivalent functional variant thereof in a sample from said patient, wherein if said yeast or mannoprotein or equivalent functional variant thereof is detected, then said patient has a high probability of suffering metastasis.

In another aspect, the invention relates to an *in vitro* method for designing a personalized therapy for a patient suffering from cancer which comprises detecting a yeast or a mannoprotein or an equivalent functional variant thereof in a sample from said patient, wherein if said yeast or mannoprotein, or functional variant thereof is detected, then the patient is a candidate to receive a treatment based on an antifungal agent or on a compound capable of binding to a mannoprotein, or to an equivalent functional variant thereof, and blocking the binding of said mannoprotein or equivalent functional variant thereof to an endothelial cell receptor.

The particular embodiments of the invention which have been discussed in the present description are applicable to the inventive aspects aimed at an *in vitro* method for predicting the probability of a patient suffering from cancer to suffer from metastasis or an *in vitro* method for designing a personalized therapy for a patient suffering from cancer.

The invention is described below by means of the following examples having an illustrative character and which in no way limit the invention.

### EXAMPLES

The materials and methods described below were used to perform the Examples 1 to 9.

### I. Material and methods

### Sourcing, maintaining and obtaining C. albicans cells

Four strains were used to carry out this invention:
- The strains *C*. *albicans* UPV1413 and *C*. *albicans* UPV1360 belonging to the culture collection of the Universidad del Pais Vasco (UPV/EHU) which were isolated from systemic candidiasis patients;
- The strain CA2, agerminative mutant kindly transferred by A Cassone (Istituto Superiore di Sanità, Rome, Italy); and
- the reference strain NCPF3153 from the National Collection of Pathogenic Fungi.

Sabouraud broth (SDB) *[20 gll glucose, 10 gll peptone],* Sabouraud glucose agar (SDA) *[11 Sabouraud broth, 15 g*/*l agar]* and 0.9% saline solution were used for maintaining the yeasts. All of them were sterilized in autoclave at 1.1 atm., 15 minutes.

The strains were maintained at 4°C in inclined tubes of SDA medium after a prior incubation of 24 hours at 24°C and were reseeded monthly in the same maintenance medium. The blastospores were obtained by means of inoculating 10⁶ cells/ml in 1 liter of SDB and it was incubated for 24 hours at 24°C. Subsequently, the cells were harvested by centrifugation for 5 minutes at 2,500 g and two washings were preformed with the Dulbecco PBS solution. 10⁶ cells/ml from the precipitate were inoculated in M199 medium and they were incubated at 37°C for 4 hours. The germ tubes thus obtained were then harvested.

The inactivation of C. *albicans* was carried out from a suspension of yeasts at a concentration of 10⁷ cells/ml which were killed by means of exposure to 20 mM or 50 mM sodium metaperiodate for 30 minutes at room temperature (Filler et al., 1996 Infect Immun.; 64:2609-2617.) or by incubation at 70°C for 30 minutes in a thermostatic bath. The viability of the cell suspension was confirmed by extension in SDA plates.

### Analysis of the wall mannosylation by means of flow cytometry.

The yeasts from the strain of *C*. *albicans* UPV1413 living or previously treated with metaperiodate at concentrations of 20 and 50 mM as has been described above were centrifuged and resuspended at a concentration of 10⁷ cells/ml in TBS (Tris-HCl (pH 7.5) 50 mM, NaCl 150 mM) + 1 mM (MgC12 + MnCl2 + CaCl2) and Concanavalin-FITC (2 µg /ml) for 30 minutes at 37°C. After the incubation, the cells were centrifuged and were resuspended in TBS to be analyzed in the flow cytometry.

The cytometry measurements were made by means of the FACSCalibur flow cytometry (Becton Dickinson). The samples were processed several times for a pre-determined time (1 minute) so that the results were repetitive and significant. The flow rate was determined before and after each session using the BD TruCOUNTTM tubes (Becton Dickinson). Subsequently, the data obtained were analyzed using the CellQuest software computer program (V 3.3; Becton Dickinson).

### B16 melanoma cells

The B16 melanoma (MB16) is a very undifferentiated malignant tumor which was isolated in the Jackson laboratories (Maine, USA) in 1954 from a spontaneous tumor emerged in the skin of a mouse of the strain C57BL/6J. In 1972, starting from this line IJ Fidler obtained cell variants with different metastatic potential by means of intravenously repeated implants of the tumor cells obtained from the pulmonary metastatic focuses. Thus a line with great metastatic capacity which is known as B16F10 was selected (Fidler, IJ. et al. 1973. Nature, 242:148-149). This tumor was provided by the American Type Culture Collection.

The B16 melanoma cells were cultured in Modified Dulbecco Eagle Medium (DMEM) supplemented with 10% fetal bovine serum, 100 units/ml of penicillin, and 100 mg/ml streptomycin. The cultures were maintained at 37°C in 5% CO2 atmosphere.

### Obtaining cells from the HSE

The liver of anesthetize mice was perfused through the vena portal with the Gey saline solution (GBSS) (pH 7.4) at 1.5 ml/minute for 3 minutes. Then, the tissue was digested by means of in situ perfusion with 10 ml of solution A *[*10 ml GBSS, 0.1% pronase*]* followed by 15 ml of solution B *[*15 ml GBSS, 0.083% (w/v) collagenase, 0.033% (w/v) pronase*].* The liver was then cut and mixed in 13 ml of solution C *[*13 ml GBSS, 0.038% collagenase, 0.038% pronase, 0.0005% (w/v) DNase I*]* at 37°C for 10 minutes. After this final enzymatic digestion the solution was diluted with GBSS and all the volume was filtered through a sterile nylon gauze. The filtrate was distributed in two 50 ml tubes and was centrifuged at 1500 rpm for 7 minutes at 4°C. A second washing was performed in the same conditions.

The cell suspension was centrifuged at 800 g for 15 minutes in metrizamide solution *[*10 ml GBSS, 17.5% metrizamide (2-(3-acetamide-5-methyl acetamide-2,4,6-triiodobenzamide )-2-deoxy-D-glucose)*]*. The cells located in the upper part of the gradient were then collected, were resuspended in DMEM-HEPES (10 mM, pH 7.4) and were centrifuged at 500 g for 7 minutes at 4°C. The cell pellet obtained was resuspended in a small volume of DMEM to calculate the proportion of viable cells by means of an exclusion test with trypan blue and counting with hemocytometer. To obtain a population of sinusoidal cells in optimum conditions, they were seeded on a 1% type I collagen matrix. For the preparation of these culture plates with this matrix 24-well plates were used to which the collagen solution was added at a ratio of 1 ml/well. After the incubation thereof at 37°C for at least 2 hours, the isolated cells were washed and were directly seeded.

The endothelial cells recently isolated in DMEM with 10% FBS were added to 24-well plates blocked with type I collagen at a ratio of 106 cells/ml per well, and they were washed after 2 hours. The resulting HSE adherent cell cultures were considered as pure because the other cell types were removed completely in the washing. The HSE cell cultures were stabilized and maintained in pyrogen-free DMEM medium supplemented with 10% fetal bovine serum, 100 units/ml of penicillin and 100 mg/ml streptomycin at 37°C in a 5% CO2 atmosphere for at least 12 hours. After culturing, the endothelial cells were washed and were maintained in medium without serum for at least 4 hours before performing any type of assay. In these conditions, the endothelial cells can be maintained in culture for 4-5 days at most, starting from which they will lose their characteristics.

### Obtaining and purifying the crude extract of C. albicans

Once the cells were harvested, these were resuspended in lysis buffer (Molloy et al. 2000. Annal Biochem, 280:1-10). Subsequently, the suspension was sonicated with pulses of 2 seconds at 60 W for 10 minutes. This process was carried out in an ice bath to prevent the heating and the subsequent degradation of the sample. The sample was then centrifuged for 10 minutes at 10,000 g to remove all the cell residues and supernatant which was called crude extract was collected.

### Purifying the mannoproteins from the crude extract of C. albicans

To separate the mannoproteins from the crude extract of *C*. *albicans,* a Con-A Sepharose affinity chromatography column which was formed by Concanavalin A (ligand) coupled to a cross-linked agarose matrix (Sepharose 4B) activated with cyanogen bromide (GE-Healthcare) was used.

The Con-A Sepharose was supplemented with 0.1 M pH 6 acetate buffer (1M NaCl, 1 mM CaCl2, 1 mM MnCl2 and 1 mM MgCl2). A solution containing 25% binding buffer *[*20 mM *Tris-HCl* (pH 7.4), 0.5 M NaCl, 1 mM MgCl2, 1 mM MnCl2, 1 mM CaCl2*]* and 75% gel (supplemented) which was poured into the column, was prepared. The volume of the bed of the column was washed 10 times with binding buffer to remove the merthiolate (0.01%), and refilling the column with binding buffer was completed and finally the upper part of the column was assembled. After achieving a constant column height, the maintenance flow was maintained at three times the volume of the bed of the column, using a flow of 0.8 ml/minute to pack the column.

A total of 90 mg of protein dissolved in 30 ml of binding buffer at a flow speed of 0.2 ml/minutes were then loaded in the column. The fraction not retained in the column was collected by passing 40 ml of binding buffer therethrough. This fraction is called protein fraction (PF). The retained fraction or mannoprotein fraction (MNF) was eluted from the column passing 40 ml of elution buffer *[*20 mM Tris-HCl (pH 7.4), 0.5 M NaCl, 1 mM MgCl2, 1 mM MnCl2, 1 mM CaCl2, 0.5 M a-D-methymannoside] therethrough.

Once the process ended, the column of Con A-Sepharose was regenerated by means of a washing with regeneration buffer *[*0.1% Triton-X 100*]* at 37°C.

### Quantifying proteins and carbohydrates

The proteins were quantified by means of the RC/DC Protein Assay Reagent
(Bio-Rad) using different concentrations of bovine serum albumin as standard line.

The sugar was quantified using the anthrone method (Chung & Nickerson, 1954. J Biol. Chem, 208: 395-407) using different concentrations of mannose to prepare the standard line.

### Preparative electrophoresis

The proteins of interest were isolated by means of the 491 Prep. Cell (Bio-Rad) and the Econo-system (Bio-Rad) the optimum conditions for the isolation thereof which are summarized in the following table being calculated for each band:

| Molecular Mass | F1 0-29 KDa | F2 29-45 KDa | F3 45-66 KDa | F4 66-116 KDa | F5 >116 KDa |
|---|---|---|---|---|---|
| % | 12% | 12% | 10% | 6% | 3% |
| Gel height | 7 cm | 7 cm | 7.5 cm | 8 cm | 8 cm |
| Elution | 0.5 ml/min | 0.5 ml/min | 0.5 ml/min | 0.5 ml/min | 0.5 ml/min |
| Fractions | 2 ml | 2 ml | 2 ml | 2 ml | 2 ml |

Table 1: Conditions used for fractioning the crude extract from the strain of *C.albicans* UPV1413 by means of preparative electrophoresis in 5 subfractions with different molecular weight.

The electrophoresis were carried out with Preparative electrophoresis buffer (10x) *[*30.3 g/l Tris base, 144 g/l glycine, 10 g/l SDS*] and* were migrated by adding 15 µl of Loading buffer *[*50 µl β-mercaptoethanol, 100 µl glycerine, Bromophenol blue traces*]* on 50 µl of sample in gel tube of 28 mm inner diameter at 12 W as limiting factor, 50 mA and 300 V. The times for obtaining the protein of interest varied from one electrophoresis to another therefore in each isolation process 80 tubes which were analyzed in fives by means of silver staining in 10% and 7.5% gels were recovered according to the molecular weight of the protein of interest to be analyzed. The analysis was performed by means of inoculating 25 ml of sample with loading buffer in each gel lane. Once the series of tubes containing the protein of interest is identified, these were analyzed one by one. The collected fractions were subsequently dialyzed against distilled water and were lyophilized.

### Preparative isoelectric focusing in the Rotofor

The crude extract was dialyzed against water and subsequently was diluted in distilled water until reaching a volume of 19 ml. 1 ml (2%) of ampholytes from the pI range in which the proteins were to be separated, was then added. To that end, two ranges, 3-10 and 3-5 were used to obtain two different fractionations. The membranes of both electrolytes were equilibrated for 24 hours in their respective buffers before performing the isoelectrophoretic separation. The cationic exchange membrane was equilibrated in the solution of acidic electrolyte *[*9.8 g/l H3PO4*]* and the anionic exchange membrane in the solution of basic electrolyte *[*4 g/l NaOH*]*. The conditions at which the preparative isoelectric focusing was performed were 12 W constant for approximately 4 hours (until the volts were stabilized). The temperature at which the process was carried out was 4°C.

### (1D) SDS-PAGE

To perform the SDS-PAGE polyacrylamide gel electrophoresis the following materials were used:
- Acrylamide/ Bis (30% T, 2.67% C): 292 g/l acrylamide, 80 g/l N'N'-bis-methylene-acrylamide (it was filtrate and stored at 4°C once prepared).
- 1.5 M (pH 8.8) Tris-HCl: 27.23 g tris(hydroxymethyl) aminomethane, 80 ml deionized water (the pH was adjusted to 8.8 with 1 N HCl. It was brought to 150 ml with deionized water and was stored at 4°C).
- 0.5 M (pH 6.8) Tris-HCl: 6 g tris(hydroxymethyl) aminomethane, 60 ml deionized water (the pH was adjusted to 6.8 with 1 N HCl. It was brought to 100 ml with deionized water and was stored at 4°C).
- Electrode buffer (pH 8.3): 3 g/l tris(hydroxymethyl) aminomethane, 14.4 g/l glycine, 1 g/l SDS.
- Stacking gel: 0.5 M pH 6.8 2.5 ml Tris-HCl, 100 µl 10% SDS, 1.3 ml Acrylamide/ Bis (stock at 30%), 6.1 ml distilled water (the gel was gently stirred and was degassed for 10 minutes at room temperature: 50 µl 10% ammonium persulfate and 10 µl TEMED were then added).
- Resolution gel (10% acrylamide): 0.5 M pH 8.8 2.5 ml Tris-HCl, 100 µl 10% SDS, 3.33 ml Acrylamide/ Bis (stock at 30%), 4.07 ml distilled water (the gel was gently stirred and was degassed for 10 minutes at room temperature. 50 µl 10% ammonium persulfate and 10 µl TEMED were then added).

The different protein extracts were analyzed by means of SDS-PAGE according to the Laemli method (1970) in the Miniprotean II system (Bio-Rad) for 45 minutes at 70 mA, 100 W and 200 V. To determine the molecular weights of the bands obtained in the electrophoresis the high molecular weight HMW standard (Sigma) depicted in Table 2 were used as molecular weight markers.

**Table 2: Molecular weight HMW markers.**

| Protein | MW |
|---|---|
| Myosine | 205 kDa |
| α-galactosidase | 116 kDa |
| Phosphorylase B | 97.4 kDa |
| Bovine albumin | 66 kDa |
| Ovalbumin | 45 kDa |
| Carbonic anhydrase | 29 kDa |

### (2D) SDS-PAGE

In the first dimension the Immobiline DryStrips isoelectric focusing gels (Amersham) with immobilized gradient of pH 4-7 of a length of 7 cm, were used.

The IPG gels were loaded with 150 µg of sample diluted in 100 µl of rehydration buffer [8 M urea, 2% (w/v) CHAPS, 2% (v/v) Pharmalytes, 2% (v/v) β-mercaptoethanol, 0.002% bromophenol blue] and were rehydrated for 12 hours in Ettan IPGphor (Amersham). The conditions are shown in Table 3.

**Table 3: conditions of isoelectric focusing.**

| Volts | Volts/hour |
|---|---|
| 500 V | 500 V/h |
| 1000 V | 1000 V/h |
| 5000 V | 15000 V/h |

After the separation by isoelectric point, two 15 minutes incubations were preformed in
equilibration buffer [6 M urea, (pH 8.8) 75 mM Tris-HCl, (87% w/w) 29.3% (v/v) glycerol, 2% (w/v) SDS, 0.002% bromophenol blue]; the first of them with dithiothreitol (1%) and the second with iodoacetamide (2%).

The proteins were separated according to their molecular weight using two systems:
- Miniprotean II (Bio-Rad) in the conditions of 70 mA, 100 W and 200 V for 1 hour. To obtain a good resolution, gels with a concentration of 10% acrylamide were used; and
- Multiphor II, for which 8 - 18 gradient gels (GE Healthcare) were used. The conditions used are shown in Table 4:

**Table 4: Conditions of electrophoresis**

| Volts | mA | W |
|---|---|---|
| 600 | 20 | 30 |
| 600 | 40 | 30 |
| 600 | 40 | 30 |

The molecular weight HMW markers (Sigma) used in the determination of the molecular weights of the bands are shown in Table 2.

### Coomassie G250 staining

Before the addition of the dye, three 5 minutes washings were preformed in distilled water. The Bio-Safe dye (Bio-Rad) based on the colloidal Coomassie blue G250 was then added for 1 hour. Once this time ended it was washed again with distilled water.

### Silver staining

Immediately after the electrophoresis the gels were treated for at least 90 minutes in fixation solution [500 ml/l ethanol, 120 ml/l acetic acid, 50 µl/l formaldehyde (before use)]. Three 20 minutes washing were then performed with 30% ethanol. After subjecting the gels to a pretreatment with sodium thiosulfate pentahydrate (0.3 g/l) for 1 minute and washing them with deionized water three times for 20 seconds each time, they were incubated for 20 minutes in the impregnation solution [0.2 g/l silver nitrate, 0.75 ml/l formaldehyde (before use)]. The gels were finally washed two times with deionized water and the developing solution was applied thereon [60 g/l sodium carbonate, 4 mg/l sodium thiosulfate (before use), 0.5 ml/l formaldehyde (before use)]. The development was detained with the stop solution [500 ml/l ethanol, 120 ml/l acetic acid].

### Detecting glycoproteins by means of Concanavalin A

The separated proteins were transferred to PVDF membranes in a NovaBlot system (GE Healthcare) in the presence of transfer buffer *[*14.41 g/l glycine, 3.025 g/l Tris, 200 ml/l methanol] for 1 hour for 1D gels and 1 hour 30 minutes for 2D gels at 400 mA and 60 V. To check that the electrotransference had been carried out correctly, the membranes were stained with a Red Ponceau solution [4 g/l Red Ponceau, 450 ml/l methanol, 100 ml/l Acetic acid] to view the proteins. The glycoproteins were detected by means of Western blot using peroxidase-bound Concanavalin A (HRP) (Sigma). After the electrotransference the membranes were washed for 1 hour with TBS buffer and were incubated with TBSL for 1 hour at 37°C. Three 10 minutes each washing were then preformed, the first two with TBS [(pH 7.5) 50 mM Tris-HCl, 150 mM NaCl] and the last one with TBS + 1 mM (MgCl2 + MnCl2 + CaCl2). After the washings, the Immobilon-P (Millipore) membranes (previously treated in an organic solvent (methanol) and subsequently in deionized water) were incubated for 1 hour at 37°C with the lectin dissolved in TBS buffer + 1 mM (MgCl2+ MnCl2 + CaCl2) at a concentration of 0.2 µg/ml. The membranes were finally washed for 10 minutes three times with TBS. The reaction was viewed by means of chemiluminescence (Millipore).

### Western Blotting

The membrane previously wetted with methanol and miliQ water was maintained for 10 minutes in TBS before starting the incubations. The free radicals were then blocked with TBSL *[*100 ml TBS, 5 g skim milk*]* for 1 hour at 37°C under stirring. It was then incubated with the primary antibody (anti-mouse IL-1β (R&D Systems)) in a 1/500 dilution in TBSL for 1 hour at 37°C. Subsequently three 5 minutes washing were preformed with TBS and it was incubated with the secondary antibody (anti-IgG-HRP) diluted 1/20000 in TBSL for 1 hour at room temperature. Finally, the membranes were washed for 10 minutes three times with TBS. The reaction was viewed by means of chemiluminescence with the Immobilon western reagent (Millipore) on Curix RP-2 film sheets (Kodak).

### Computer analysis of images

The computer analysis of the electrophoresis of the different purified fractions was performed with the ImageMaster 2D Platinum computer program (GE Healthcare) from gels stained with Coomassie blue G250, with silver staining or developed with chemiluminescence.

### Identifying the MNF3 mannoproteins

The mannoproteins from the MNF3 (45-66 kDa) were identified by means of the MALDI-TOF MS peptide fingerprint technique in the CIC Biogune proteomic service (Zamudio) or by means of LC-MS/MS performed in the general proteomic service of the Universidad del Pais Vasco (UPV/EHU). The points were extracted from a polyacrylamide gel in 2D stained with Coomassie blue G250. The spectra obtained were analyzed by means of the GenBank and Matrix Science databases.

### Tumor adhesion assay for C. albicans-induced tumor adhesion

The adhesion was measured using the method based on the fluorescence measurement. The MB16 cells were resuspended in a solution of 2',7'-bis-(2-carboxyethyl)-5.6-carboxyfluorescein acetoxymethyl ester (BCECF-AM) in DMEM-HEPES (40 µg/ml) and were incubated for 20 minutes at 37°C. The cells were then washed and resuspended in DMEM-HEPES at a concentration of 2 x 10⁵ cells/ml. The autofluorescence of the HSE culture was determined.

Subsequently, the HSE cells were incubated with different strains and extracts of *C*. *albicans* (5 x 10⁵ cells/well), MNF (2 µg carbohydrate/ml), EC (2 µg carbohydrate/ml), PF (2 µg protein/ml) or commercial mannan from *Saccharomyces cerevisiae* (100 µg/ml) for 8 hours. The non-treated HSE cells received basal medium as control. After being washed extensively, the MB16cells labeled with BCECF were added to the HSE culture (0.1 ml/well) and also to plastic or to control wells coated with type I collagen.

Once the time of adhesion ended, the co-cultures were excited with light of wavelength 488 nm and the fluorescence emitted at 530 nm by the BCEF incorporated to the tumor cells in the scanning fluorometer in plate was recorded. Three vigorous washings of said co-cultures were then preformed, removing the tumor cells not adhered to the endothelial cell monolayer. Subsequently, 1 ml of the same adhesion medium was added in each well and the fluorescence emitted was read. Thus a relative value (in arbitrary units of fluorescence intensity) could be determined with respect to the number of tumor cells added. The relative adhesion was calculated for each well according to the formula: (A - C)/(B - C), wherein A is the fluorescence of the MB16 adhered to the incubated *C*. *albicans* HSE, B is the fluorescence of the MB16 adhered to the non-treated HSE cells and C is the fluorescence prior to the adhesion of tumor cells.

To block the IL-1β receptor or the mannose receptor, the IL-1 antagonist receptor (IL-1Ra) (Amgen Biologicals) and the anti-mannose receptor (anti mouse CD206) (Acris antibodies) were used. These molecules were added 15 (IL-1Ra) or 30 (anti- MNR) minutes before incubating the endothelium with the *C.albicans* cells or extracts.

### Measuring the B16 melanoma cell proliferation in response to C. albicans

2,550 cells/well (200 µl) were seeded in culture medium with 10% FBS *[*1 ml acetic acid, 99 ml distilled water, 0.4 mg sulforhodamine]. The standard line relating the number of cells with the amount of fluorescence emitted was made from the serial dilution of the MB16 from a concentration of 675 up to 80,000 cells/well (200 µl) in a 96-well plate. Culture medium without cells was used to measure the blank. After three hours, when the cells were adhered to the plate, 50 µl of 64% acetic acid were added on the wells which made up the standard line. After 1 hour of incubation, the wells were washed with PBS and were left to dry.

On the following day the medium with FBS was removed fresh medium without FBS being added together with the different treatments (MNF at a concentration of 2 µg carbohydrates/ml). The plate was incubated for 48 hours at 37°C with 5% of CO2. Once the time of incubation ended, 50 µl of 64% trichloroacetic acid was added on the wells for 1 hour. They were then washed with tap water and were left to dry in the stove at 50 °C for 30 minutes. 100 µl/well of sulforhodamine solution were then added for 30 minutes and was incubated at room temperature in darkness. The wells were then washed with 1% acetic acid and the plate was dried again in the stove at 50 °C for 30 minutes. Finally, 200 µl of the 10 mM pH 10.5 Tris solution *[*Tris 1.21 g/ml*]* were added per well and after 5 minutes the plate was read in a scanning fluorometer (Fluoroscan AFCENT) with a 530 nm excitation filter and 620 nm emission filter.

A regression line was constructed with the fluorescence values obtained in the standard line, an equation which allowed relating the fluorescence emitted with the cells according to the number of cells/well being obtained.

### Quantifying TNF-α

The concentration of TNF-α and IL-18 in the supernatants was determined using a monoclonal antibody-based commercial ELISA kit (R&D Systems) following the manufacturer's instructions. The tumor adhesion assays of *C. albicans-induced* tumor adhesion and B16 melanoma tumor cell proliferation assays in response to *C. albicans* (both described above) were performed in collaboration with the company, Pharmakine S.L.

### Measuring the concentration of endotoxins

All the processes for obtaining cells or cell extracts performed for the purpose of confirming the increase of the tumor adhesion induced were performed with sterile endotoxin-free material. To confirm the absence of these in the final extracts the *Limulus* lysis assay (Associates of Cape Cod, Woods Hole, MA) was used following the manufacturer's instructions.

### Statistical analyses

The statistically significant differences (p < 0.05) were calculated according to the student's t-test or by means of the ANOVA test followed by the Bonferroni post-hoc test as shown in the drawings.

### II. RESULTS

### Example 1

### Effect of Candida albicans in the tumor adhesion to HSE

### 1. Effect of the intraspecific variation and of the morphological state of different strains of C. albicans in the adhesion of the MB16 on the HSE

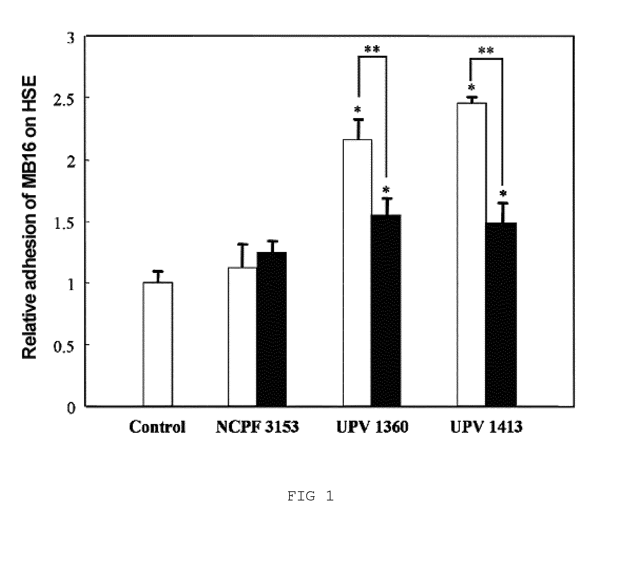

In order to clarify the effect exerted by *C. albicans* in the tumor adhesion to the mouse hepatic sinusoidal endothelium, different strains and morphological states of the same were studied. As can be observed in Figure 1, three strains were used both in yeast-like state and after having induced the formation of germ tubes: The reference strain NCPF3153 from a culture collection type did not show significant effect with respect to the control situation. Contrarily, the two strains from the collection of the Universidad del Pais Vasco which were isolated from candidiasis patients demonstrated a large capacity to stimulate the HSE and to increase the adhesion of tumor cells.

Both strains, when they were used in yeast-like state induced an increase of the adhesion of the MB16 cells to the HSE which exceeded by more than 100% the adhesion produced in the control situation, i.e., without *C. albicans.* The results also showed that after the incubation of the endothelium with the yeasts, more than 85% of them had filamented. However, when the HSE was incubated with the strains in germ tube morphology, germinated before the assay, the increase of the adhesion was significantly less than that induced by the same strains in yeast-like state, although it also was significantly greater than that of the control situation.

Since the morphological state of *C. albicans* influenced the studied model and that the yeasts were those that greatly induced the increase of the adhesion, whether this proadhesive effect induced by *C. albicans* is also depending on the yeast viability was then checked. To that end, cells, viable and killed by heat and by chemical oxidation with metaperiodate were used. This point is particularly interesting if taking into account that the yeasts germinate during the incubation with the HSE and that if this process is the determining process, the dead yeasts would not be capable of increasing the tumor adhesion to the HSE.

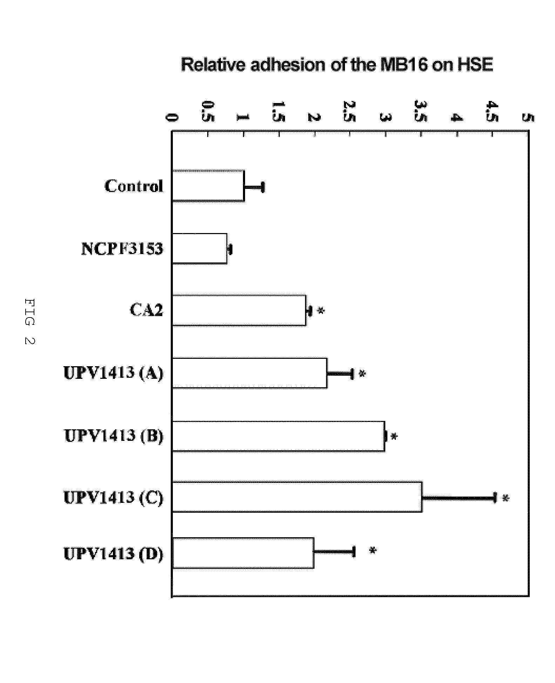

Figure 2 shows how, despite being dead, the yeasts of the strain UPV1413 continue to cause a significant increase of the adhesion of the B16 cells on the HSE. The increase is more than the double of the tumor cells adhered if it is compared with the control situation. Specifically, the cells killed both with heat and with metaperiodate at a concentration of 20 mM induce an increase of the tumor adhesion of more than 100% in comparison with the control, being higher than that produced by the living cells. This implies that neither the cell viability nor the germinative process are crucial to induce an increase of the tumor adhesion to the HSE.

Despite the fact that the cells which were treated with metaperiodate at a concentration of 50 mM reduced their effect in the increase of the tumor adhesion with respect to those treated with a concentration of 20 mM, it calls attention to the fact that the yeasts killed by means of the treatment with metaperiodate in both concentrations maintained the capacity for increasing the tumor adhesion to the endothelium, since this treatment oxidizes the sugar and is commonly used to remove a good part of them from the cell extracts.

To verify that the yeasts are the most active morphological state in this process, the strain NCPF3153 which had not shown any influence on the tumor adhesion and its agerminative mutant CA2 was tested. Figure 2 clearly shows that although the strain CA2, which is not capable of germinating, was capable of inducing a significant increase of the adhesion of MB16 cells on the HSE. This increase involved almost the double of tumor cells bound with respect to the control situation whereas the reference strain had no effect.

The incubation of the HSE with *C. albicans* causes a greater adhesion of the MB16 cells to the endothelium. This increase depends both on the strain used, and on the morphological state thereof, the morphology being yeast morphology both in living and dead states, which demonstrated greater proadhesive capacity.

### 2. Effect of the chemical oxidation with metaperiodate on the morpohology and the composition of the C. albicans wall

A flow cytometry was used to confirm that the treatment of the *C. albicans* yeasts with metaperiodate removed the carbohydrates from the cell surface. Flow cytometry (FCM) is a multiparametric cell analysis technique the principle of which is based on passing a suspension of aligned particles (generally cells) one by one by a focused laser beam. The light called FSC *(Forward* Scatter) scattered in small conical angle is a measurement proportional to the size of the particle producing the dispersion. The light called SSC *(Side Scatter)* scattered in right angle is proportional to the complexity of the inner structure of the particle. The parameter FL1 is proportional to the fluorescence of the particle, in this case due to the FITC.

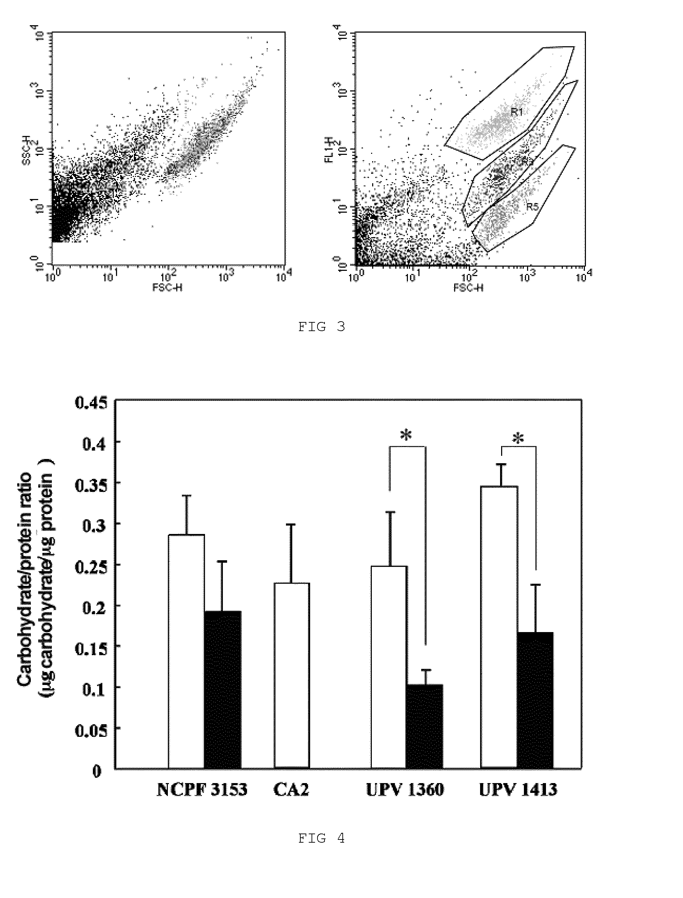

To conduct the experiment, the *C. albicans* yeasts treated with 20 mM metaperiodate, 50 mM metaperiodate and the non-treated yeasts were labelled with the lectin Concanavalin A, which has affinity to the a-mannosylated residues, bound to the fluorochrome FITC (Con A-FITC). Figure 3 shows a scatter cytogram wherein the cellular complexity (SSC) are compared with the size (FSC), and a mix cryptogram comparing the fluorescence (FL1) with the cell size (FSC). As can be observed, the different populations did not vary in term of size or cellular complexity, however, they did vary in terms of fluorescence. The living population not treated with metaperiodate (R1) has a high fluorescence as a consequence of the fact that the Concanavalin AFITC has bound thereto. However, as the concentration of the treatment with metaperiodate increases, the binding of the Concanavalin A becomes more difficult and the fluorescence reduces. Thus, the population R2 treated with 20 mM of metaperiodate, appears with lower fluorescence than the living populations, and the R3, treated with 50 mM, virtually lacks fluorescence as it shows the same fluorescence as the background noise.

Conclusion: The treatment of the *C. albicans* yeasts with metaperiodate removes the sugar from the cell surface preventing the binding of the Concanavalin A. Despite that, the yeasts maintain capacity of increasing the tumor adhesion to the endothelium with respect to the control.

### 3. Study of the carbohydrate/protein composition of the strains of Candida albicans

The differences in the concentration of proteins and carbohydrates between the strains could provide an explanation about which elements of *C. albicans* are those primarily responsible for their effect in the tumor adhesion to the HSE. To confirm the same, carbohydrate and protein measurements were performed for each of the strains in yeast and germ tube morphology .

**Table 4**

| | Protein | | Sugar | | Sugar/Protein | |
|---|---|---|---|---|---|---|
| Strain | Mean | Dev. | Mean | Dev. | Mean | Dev. |
| NCPF3153 | 7.01 | 1.74 | 1.95 | 0.23 | 0.29 | 0.05 |
| UPV1360 | 6.69 | 1.72 | 1.58 | 0.20 | 0.25 | 0.07 |
| UPV1413 | 6.52 | 0.97 | 2.23 | 0.31 | 0.34 | 0.03 |
| CA2 | 5.88 | 2.06 | 1.23 | 0.21 | 0.23 | 0.07 |
| NCPF3153 (germinated) | 10.31 | 4.78 | 2.22 | 0.47 | 0.19 | 0.06 |
| UPV1360 (germinated) | 13.38 | 3.82 | 1.50 | 0.44 | 0.10 | 0.02 |
| UPV1413 (germinated) | 9.98 | 4.90 | 1.64 | 0.35 | 0.17 | 0.06 |

As is observed in Table 4, the measurement of the concentration of carbohydrates per cell unit among the strains did not indicate differences that could be related with the different effects shown in the tumor adhesion. However, the concentration of proteins increased when the cell unit was the germ tube and therefore had a greater biomass than the yeast. This means that the carbohydrate/protein ratio is higher in yeast than in germ tube, and that this difference is significant in some cases. This difference is curiously significant in the strain UPV1413 and in UPV1360 which are those that have shown the greatest effect in the tumor adhesion to the endothelial cells (Figure 4).

Conclusion: The carbohydrate/protein ratio is higher in the blastoconidia of all the strains of *C. albicans* with respect to the germ tubes, this difference being significant in the strains UPV1360 and UPV1413.

### Example 2

### Effect of Candida albicans in the production of IL-18

The IL-18 has been described as a molecule responsible for increasing the adhesion of the MB16 cells to the murine hepatic endothelium and for increasing the capacity of metastasis of MB16 in mouse liver through the stimulation of the molecule VCAM-1 (Carrascal et al., 2003. Cancer Research, 63: 49731-49734). For this reason, to confirm that it is also one of the cytokines that takes part in the process triggered by *C. albicans,* the production of this cytokine after the incubation of the HSE with living and dead yeasts was quantified by means of oxidation with metaperiodate at two different concentrations: 20 and 50 mM.

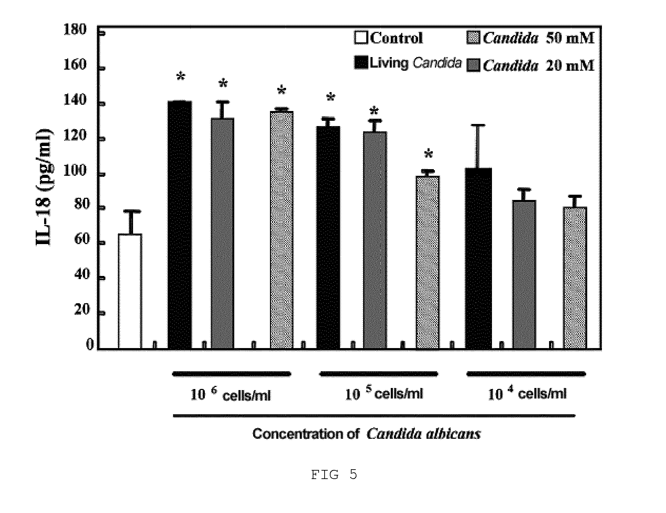

As can be observed in Figure 5, *C. albicans* at a concentration of 106 cells/ml induces an increase of the production of IL-18 of approximately 100% with respect to the values of the control situation regardless of whether the yeasts are living or dead. The significant increase of the production of IL-18 is being maintained despite the fact that the concentration of yeasts is reduced to 105 cells/ml. In this concentration however, it can be observed that the yeasts killed with 50 mM metaperiodate have a lower effect than the living yeasts and that the yeasts killed with a concentration of 20 mM, clearly showing the importance of completely removing the carbohydrates from the *C. albicans* cell surface.

Conclusion: *C. albicans* induces the production of IL-18 in the HSE. This production is regardless of the concentration of cells, as well as of the concentration of carbohydrates present in the yeast wall.

### Example 3

### Study of the effect of the different fractions of C. albicans in the tumor adhesion to the HSE

### 1. Purification of the protein and mannoprotein fraction

Subsequently, since an effect in the increase of the adhesion of tumor cells to the HSE induced by commercial mannan (Mendoza .et al. 1998. J. Cell Physiol., 174: 322-330) has been described in the literature, and that the strains used in this study are rich in this mannose polymer, whether the mannoproteins of the strain which had demonstrated the greatest effect, the strain UPV1413, were those responsible for this response was analyzed. To that end, an affinity chromatography in Concanavalin A-Sepharose 4B columns was performed to separate the mannoprotein fraction (MNF) from the non-mannoprotein fraction called protein fraction (PF).

The results obtained which are reflected in Table 6 show a not very high yield from the chromatography. However, taking into account that the results that appear in the literature with this type of columns speak of yields of 60% (Rodrigo et al. 2003. Biochem J., 376:261-268) in specific proteins and that the extract of the present invention is very complex, the results can be said to be satisfactory. The MNF obtained had an approximated concentration of 45 - 69% of carbohydrates. In other words, the mannoprotein fraction obtained from *C. albicans* has a concentration of carbohydrates slightly higher than that of proteins.

**Table 6: Results of affinity chromatography in Concanavalin A columns which are shown as the mean of the chromatography results and their columns standard deviation.**

| Fraction | Protein (mg/ml) | Dev. | MN (mg/ml) | Dev. |
|---|---|---|---|---|
| Crude | 26.08 | 3.88 | 8.92 | 1.24 |
| PF | 16.08 | 2.95 | 0.35 | 0.46 |
| MNF | 3.3 | 0.98 | 4.49 | 0.99 |
| Yield (%) | 74.31 | | 54.26 | |

### 2. Effect of the concentration of the fractions of C. albicans in the tumor adhesion to the HSE

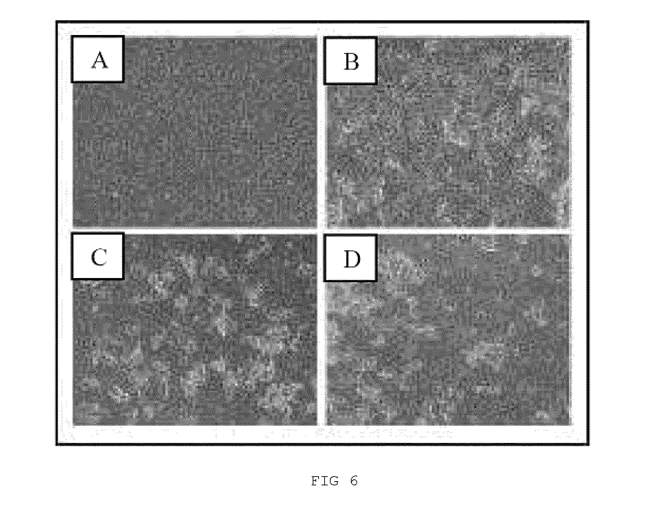

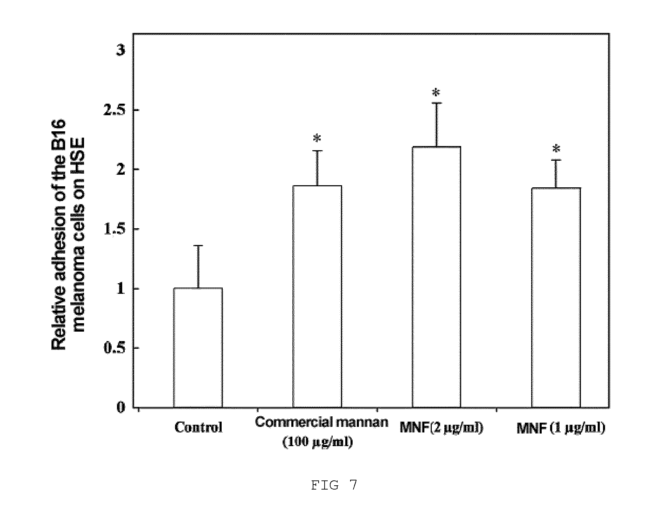

Once the fractions are obtained, they were contacted with the endothelium cells and it was observed that at concentrations similar to those tested in the literature with commercial mannan (0.1 mg/ml), the fractions obtained in the current laboratory were toxic for the cells (Figure 6). To enable confirming the effect of the different fractions obtained in the adhesion of the MB16 to the HSE the samples must be diluted to 2 µg/ml and even to 1 µg/ml. At this concentration, the HSE cells were not damage and the increase of the tumor adhesion was similar to that of the 100 time more concentrated commercial mannan (0.1 mg/ml). The effect observed by using a concentration of mannoproteins of 2 µg/ml exceeded by more than double, i.e., more than 100%, the increase of the tumor adhesion with respect to the control situation (Figure 7). This increase is similar to that obtained after stimulating the endothelium with the living yeasts of this strain. 3. Effect of the different fractions of *C. albicans* in the tumor adhesion to the HSE

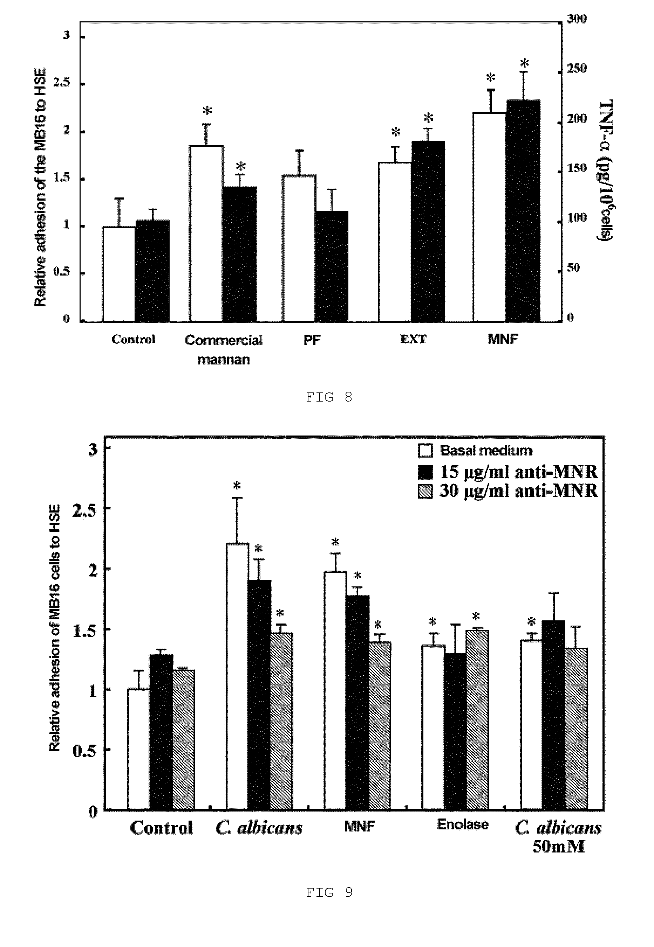

The following point to be studied was whether the increase of the number of tumor cells adhered to the endothelium, as a consequence of having been in contact with *C. albicans,* was only due to the mannoproteins thereof. To that end, the effect of the MNF, the PF and crude extract in increasing the adhesion of the MB16 to the HSE was compared. As can be observed in Figure 8, in the HSE stimulated with the MNF an increase of the adhesion of the MB16 significantly greater than that of the control was produced, again inducing an increase greater than 100%. However, the PF did not induce a significant increase of the tumor adhesion. The crude extract did slightly increase the tumor adhesion, although the effect was lower than that produced by the MNF.

### 4. Effect of the different fractions of C. albicans in the production of TNF-α

The stimulation of the endothelium causes a cascade of cytokine which ends with the expression of adhesion molecules such as the VCAM-1 (Mendoza (cited *at supra)* 1998, Vidal-Vanaclocha et al. 2000. PNAS, 97: 734-739) wherein the tumor cells are adhered. To know whether the process triggered by the MNF which as a consequence caused the increase of the adhesion of MB16 cells to the HSE was also mediated by cytokines, the concentration of TNF-α, the cytokine which is described as one of the first and most important cytokines acting in the proinflammatory cascade described in the HSE was measured.

As shown in Figure 8, the PF was not capable of stimulating an increase of the production of TNF-α in the endothelium with respect to the control. However, the incubation of the endothelium both with the crude extract and with the MNF did induced a significant increase in the production of TNF-α compared with the control situation. Again, similarly to that which occurred with the increase of the tumor adhesion, the MNF showed a greater capacity of inducing the production of TNF-α than the crude extract since this did not induce an increase of 100% whereas the stimulation with the MNF produced approximately a 120% increase of production of TNF-α with respect to the control, reaching a values of 220 pg/106 cells.

Conclusion: The mannoprotein fraction of *C. albicans* induces the adhesion of tumor cells to the HSE at a concentration 100 times more diluted than the commercial mannan. In this response TNF-α is produced and it is toxic at high concentrations of extract. Both the tumor adhesion and the TNF-α increase by more than 100% with respect to the control when the HSE is stimulated with the MNF.

### Example 4

### Effect of the mannose receptor in the tumor adhesion to the HSE and the production of IL-18

### 1. Effect of the mannose receptor in the tumor adhesion to the HSE

In order to demonstrate whether the increase of the tumor adhesion to the endothelium stimulated by the mannoproteins of *C. albicans* was completely mediated in the starting point thereof by the mannose receptor, a monoclonal antibody capable of blocking the binding between the mannose receptor and its ligands was used. Therefore, a complete inhibition of the increase of the tumor adhesion mediated by the mannoproteins of *C. albicans* would imply that all the response caused by *C. albicans* entailed at the start thereof an activation of said receptor.

In Figure 9 it can be observed that the use of the anti-mannose receptor considerably reduced the increase of the tumor adhesion to the endothelium stimulated by *C. albicans* or its mannoproteins. As the concentration of this antibody increased, the adhesion was increasingly reduced, although the effect was not completely eliminated. In fact, it was confirmed that the increase of the tumor adhesion induced by the recombinant enolase, which was approximately 40%, was, as was expected, independent of the use of the anti-mannose receptor since it was a recombinant enolase expressed in *E. coli* which does not have adhered carbohydrates. This involves the activation of the process by another mechanism independent of this receptor. Furthermore, *C. albicans* treated with 50 mM of sodium metaperiodate which removes the carbohydrates from the cell surface, increases the tumor adhesion approximately 40% with respect to the control. This effect is maintained, although in a non-significant manner, despite adding the anti-mannose receptor.

Conclusion: The mannose receptor is the one responsible for starting most of the response of the HSE against *C. albicans* triggering an increase of the tumor adhesion, but there is also an effect independent of the same.

### 2. Effect of the mannose receptor in the production of IL-18

As has been mentioned previously in this invention, the IL-18 has been described as a molecule responsible for increasing the adhesion of MB16 cells to the murine hepatic endothelium in the inflammatory process started after the stimulation of the mannose receptor which ends with the expression of the VCAM molecule where the tumor cells are bound. Thus, to further clarify the effect of the mannose receptor in this process, the production of IL-18 by the HSE stimulated with *C. albicans* and its mannoproteins was studied, and the mannose receptor was blocked to check its influence in the production of this cytokine.

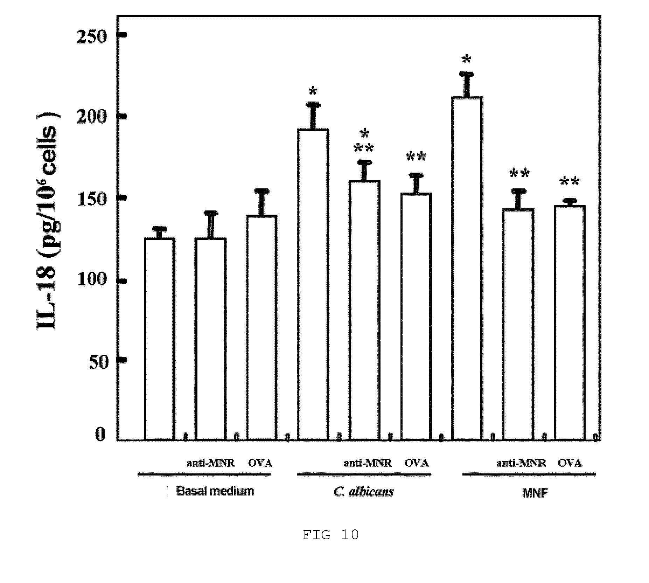

As observed in Figure 10, the production of IL-18 induced by *C. albicans* or its mannoproteins reduced significantly by using the anti-mannose receptor. The same happened when the ovalbumin, a glycoprotein blocking the binding sites to the endothelium both with its glycidic and protein parts, was used. As observed in the drawing, the reduction of the production of IL-18 between 45 and 83% of inhibition of the increase of the production of IL-18 induced by *C. albicans* and its mannoproteins in the endothelium was significant. After the blocking with both molecules the production of IL-18 is greater than the control situation being significant in the endothelium stimulated with *C. albicans* and blocked with the anti-MNR.

Conclusion: The anti-mannose receptor antibody significantly reduces the production of IL-18 by the HSE stimulated by *C. albicans.*

### Example 5

### Two-dimensional study of the crude extract, protein fraction and mannoprotein fraction of C. albicans

In order to view and characterize the peptide composition of the fractions obtained, the same was separated by means of two-dimensional electrophoresis and then the gels obtained were stained by means of silver staining because it is more sensitive than the staining with Coomassie blue and it better stains the mannoproteins. The figures show that the crude extract has proteins distributed throughout the isoelectric point range comprised between 4 and 8, whereas by molecular weight the proteins having a size greater than 100 kDa were not detected.

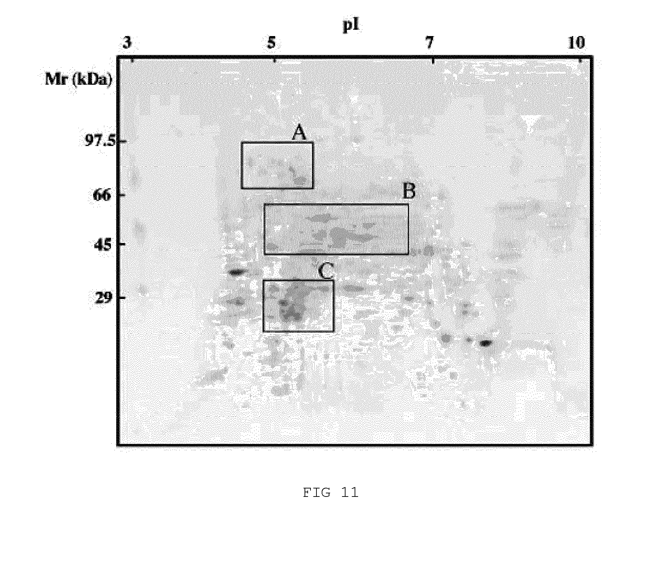

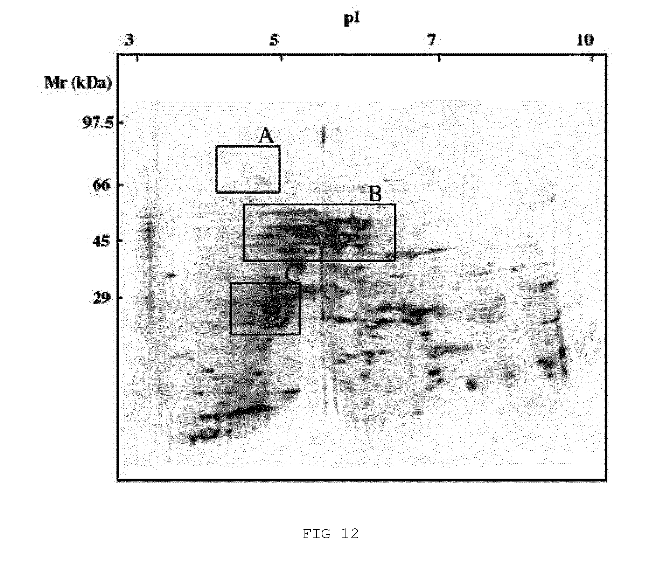

The greatest molecule density was located between 4.5 and 7 isoelectric point and between 40 and 60 kDa molecular weight (Figure 11(B)). Two significant areas both above 66 kDa and in 27-30 kDa were also observed. Both with approximate isoelectric points of 5 (Figure 11 (A and C)). Figure 12 shows the result of the two-dimensional electrophoresis of the PF. As can be observed therein, most of the molecules that appeared in gel of the crude extract belong to this fraction, identifying the proteins which still do no appear being virtually impossible due to the high density of contained proteins. However, the area called A which is located between 65 -75 kDa and 4.5-5 isoelectric point which has almost completely dissapeared can be exempted.

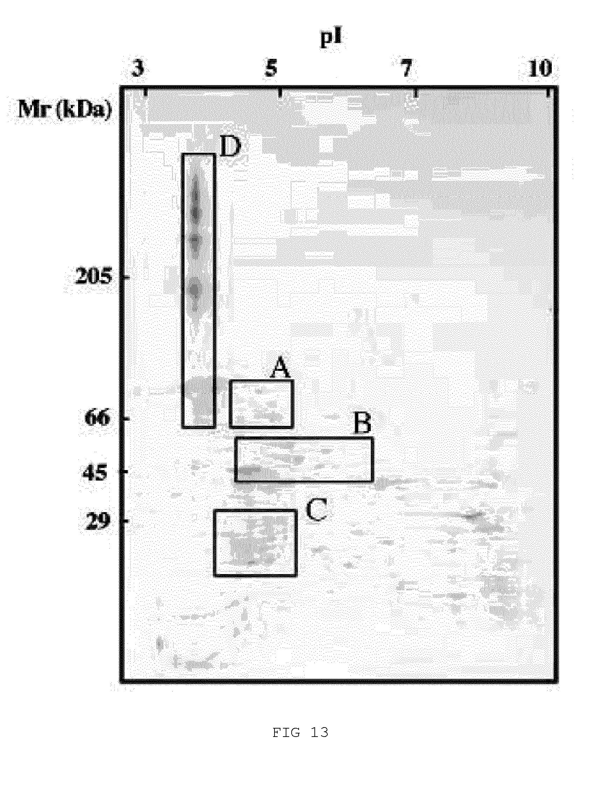

As has been observed in Table 6, the amount of proteins containing the MNF was approximately 5 times lower than the amount which was in the PF. This meant that to achieve gels of the MNF in which the mannoproteins thereof could be viewed, a 5 times higher proportion of sample had to be added. To that end, the mannoproteins appearing in the gel of MNF are not visible in the gel of the crude extract because their concentration is 5 times lower. The MNF shown in Figure 13 has a distribution much less homogenous than the other fractions. It was observed that the number of mannoproteins in relation to the total is small and that they are practically distributed entirely in the 3 areas mentioned above. Area A which is mostly formed by mannoproteins and which as can be seen in the drawing, is one of the most significant area of the MNF stands out in this case.

Additionally, it was interesting to observe how an acidic, electron-dense and very high molecular weight area which even exceeds 200 kDa and which could not be observed in the electrophoresis performed with the crude extract (Figure 13 (D)) appeared in the gels made with the MNF.

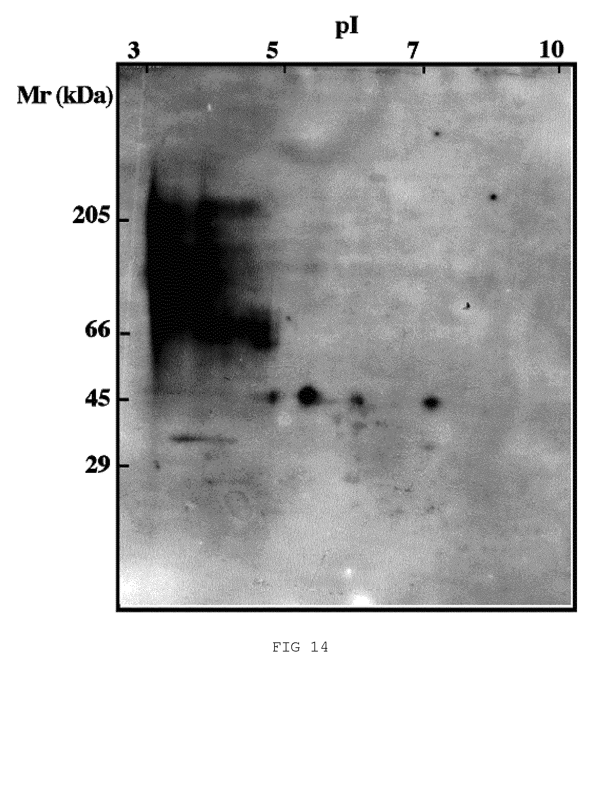

A two-dimensional Western blot of the crude extract developed with chemiluminescence using Con A-HRP (Figure 14) shows the high degree of mannosylation of the high molecular weight acidic mannoproteins which could not be detected in the silver staining of the crude extract but were detected in the gel of the MNF and were called as area D. There are also several highly mannosylated mannoproteins with a molecular weight of approximately 45 kDa. At lower molecular weights, the low degree of mannosylation did not allow them to be detected.

Conclusion: Most of the proteins from the extract of *C. albicans* viewed by means of two-dimensional electrophoresis are not mannosylated. To enable viewing the mannoproteins by means of this methodology a prior purification such as the one performed in the present invention is necessary.

### Example 6

### Analysis of mannoprotein subfractions in the tumor adhesion to the HSE

Once demonstrated that the mannoprotein fraction has an important role in increasing the tumor adhesion to the HSE induced by *C. albicans* and that this process is produced by means of activating a cascade of cytokines among which include TNF-α and IL-18, the following objective that was proposed was to obtain different mannoprotein subfractions covering different molecular weights and different isoelectric points. Therefore, the effect of this fraction was further specified. In other words, whether its effect is generalized or is concentrated in some mannoproteins.

### 1. Obtaining mannoprotein subfractions of different molecular weight

The results obtained from the purifications by means of preparative electrophoresis which appear in Table 7 shown a total yield of the process ranging from 51.25% which was obtained if proteins are taken into account up to 80.53% which was obtained for the carbohydrates. This difference could be due to the fact that the most difficult to recover fractions, the central fractions, is where most amount was lost since the first and the last tubes of the fractions had to be rejected because they were contaminated with the immediately adjacent ones, and the protein amount was greater than the mannoprotein in these fractions. After the separation of the crude extract into 5 subfractions of different molecular weight, these were subjected to an affinity chromatography in a Concanavalin A-Sepharose column as has been described above to purify the MNF.

**Table 7: Results from obtaining 5 subfractions of different molecular weight by means of preparative electrophoresis from the crude extract: F1: 0 - 30 kDa, F2: 30 - 45 kDa, F3: 45 - 66 kDa, F4: 66 - 115 kDa, F5: <115 kDa which is expressed as the mean of the results obtained, their standard deviation and the certain percentage of the total crude extract representing the amount collected from each fraction.**

| Fraction | Starting protein (mg) | Ending protein (mg) | Dev. | Protein (%) | Starting MN (mg) | Ending MN (mg) | Dev. | MN (%) |
|---|---|---|---|---|---|---|---|---|
| F1 | 40 | 9.37 | 0.75 | 23.43 | 12 | 1.03 | 0.01 | 8.58 |
| F2 | 40 | 4.22 | 0.72 | 10.54 | 12 | 1.41 | 0.27 | 11.77 |
| F3 | 40 | 2.66 | 1.16 | 6.64 | 12 | 1.81 | 0.92 | 15.12 |
| F4 | 40 | 3.19 | 0.60 | 7.98 | 12 | 2.59 | 0.21 | 21.58 |
| F5 | 40 | 1.06 | 0.07 | 2.66 | 12 | 2.82 | 0.07 | 23.48 |

**Table 8: Results from obtaining the 5 mannoprotein subfractions obtained by means of affinity chromatography from those obtained by means of preparative electrophoresis from the crude extract: MNF1: 0 - 30 kDa, MNF2: 30 - 45 kDa, MNF3: 45 - 66 kDa, MNF4: 66 - 115 kDa, MNF5: <115 kDa which is expressed as the mean of the results obtained, their standard deviation and the yield in certain percentage of the chromatography.**

| Fraction | Starting protein (mg) | Ending protein (mg) | Dev. | Protein (%) | Starting MN (mg) | Ending MN (mg) | Dev. | MN (%) |
|---|---|---|---|---|---|---|---|---|
| MNF1 | 32.62 | 2.50 | 0.82 | 7.63 | 5 | 1.01 | 0.51 | 20.20 |
| MNF2 | 13.82 | 2.50 | 0.65 | 10.66 | 5 | 1.40 | 0.09 | 27.92 |
| MNF3 | 7.21 | 1.43 | 0.06 | 11.91 | 5 | 1.65 | 0.68 | 32.93 |
| MNF4 | 5.83 | 1.23 | 0.21 | 25.17 | 5 | 1.70 | 0.49 | 33.90 |
| MNF5 | 2.21 | 1.04 | 0.09 | 0.80 | 5 | 1.78 | 0.20 | 35.58 |

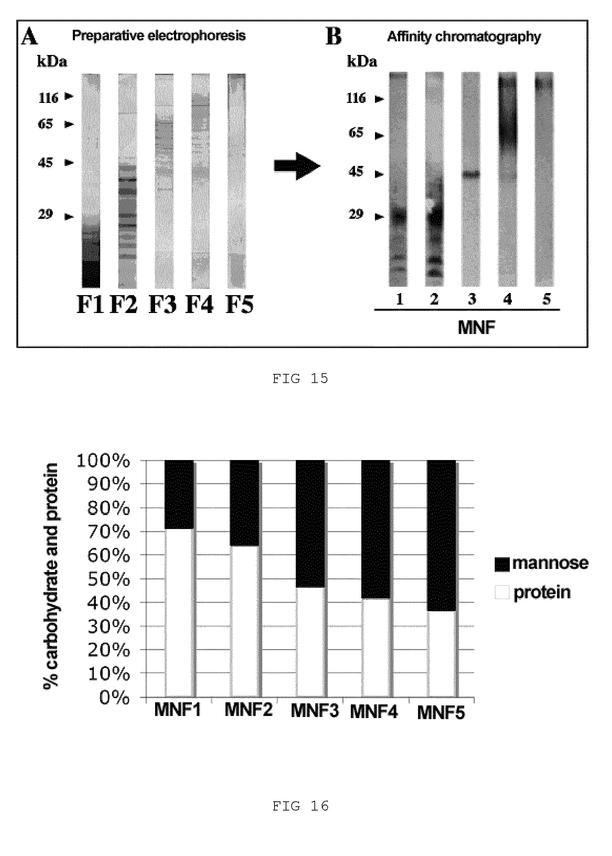

The results of the purification obtained by means of affinity chromatography of the fractions separated according to their molecular weight to obtain their respective mannoprotein fractions showed, as seen in Table 8, that the yields were lower than those obtained when purifications were performed from the total crude extract. This could be due to the fact that the amount from which it started was lower, and therefore any loss produced throughout the purification or in the subsequent dialysis and lyophilization thereof before the measurement represented a greater percentage of loss. Curiously lower yields were further obtained when the molecular weight and the proportion of mannose/protein of the fraction was lower. Figure 15 shows the result of the fractions obtained after the separation by molecular weight of the crude extract (A) where an excellent separation between the fractions was confirmed. Furthermore, the Western Blot which was performed after the purification by means of affinity chromatography continues to show a very good separation between the fractions. In both cases only fraction 2 partly overlapped fraction 1.

Figure 16 depicts the carbohydrate/protein proportion of each of the fractions obtained. It demonstrates that the mannoproteins of higher molecular weight are most mannosylated. It is observed that both the MNF1 and the MNF2 have a greater proportion of protein than of carbohydrates, whereas the situation is reversed in the fractions MNF4 and MNF5. The MNF3 has a proportion of carbohydrate more or less equal to that of protein.

### 2. Effect of the different mannoprotein subfractions separated according to their molecular weight in the tumor adhesion to the hepatic sinusoidal endothelium

Once the correct separation of the mannoprotein fraction into 5 subfractions of different molecular weights is performed, the effect of each of them in the tumor adhesion to the endothelium was confirmed. To that end, for the purpose of preventing that the difference between the effect of the different subfractions was due to the amount of carbohydrate present in each fraction, the concentration of the 5 subfractions was adjusted to the same concentration of carbohydrates (2 µg/ml).

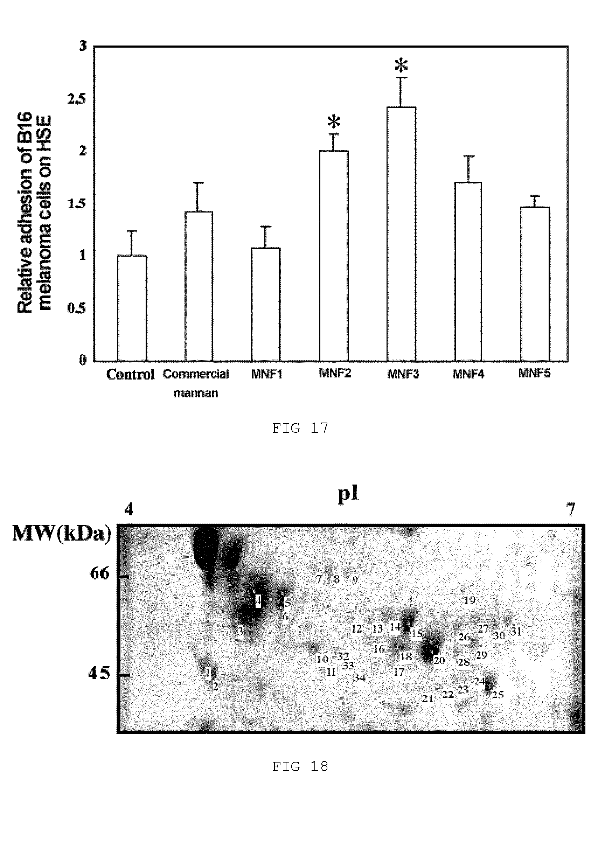

After the incubation of the hepatic sinusoidal endothelium with each of the subfractions (Figure 17), the increase of the tumor adhesion was significantly greater than in the control situation in 4 of the 5 subfractions (MNF2, MNF3, MNF4 and MNF5), both MNF2 (30 - 45 kDa), and MNF3 (45 - 66 kDa) being those which had the most effect reaching increases of approximately 100% and even reaching 150% in the case of the MNF3.

Furthermore, as can be observed in Figure 17, the MNF2 and MNF3 induced a significant increase of the tumor adhesion, of 35 and 60% respectively, with respect to the commercial mannan, the latter having a concentration of carbohydrates 50 times higher.

### 3. Sequencing the MNF3

According to the results obtained, there were differences between the fractions of different molecular weight that had nothing to do with the concentration of carbohydrates. The MNF3 specifically showed a very significant and greater effect than the rest of the mannoprotein fractions obtained.

For this reason, the most abundant mannoproteins of the MNF3 was identified. To that end, two-dimensional electrophoresis which were stained with Coomassie blue and from where the most representative mannoproteins were subsequently identified, were performed. For the identification an analysis was performed by means of peptide fingerprint of the points which were the most intense and repetitive in 2DE gels. Figure 18 shows the points of the MNF3 which were selected for their identification. They were manually extracted and they were identified in the CIC Biogune (Zamudio).

The identification of the 34 points which are labeled in Figure 18 gave as a result a list of 15 different mannoproteins forming the MNF3 (Table 9).

All of them were identified with a significant reliability if taking into account their score and the ratio of the number of peptides detected among the number of total peptides of the protein (columns 6 and 7 respectively) with the exception of point number 3, the zinc protein (Zinc Ring finger protein), the identification of which was rejected because it gave a very low score and few peptides were detected.

Among the mannoproteins identified many of them were repeated in contiguous isoforms with the same molecular weight but with different isoelectric points such as in the case of the aminopeptidase, the fructose bisphosphate aldolase, the alcohol dehydrogenase or the enolase. The last two also have subunits in which both the molecular weight and the isoelectric point varied. This was also the case of, for example, the protein disulfide isomerase. Many of the sequenced mannoproteins have been previously described in the literature as antigens. Such is the case of the mitochondrial ketol acid-reductoisomerase, the protein disulfide isomerase, the enolase or the alcohol dehydrogenase (Pitarch et al., 2004. Proteomics, 4: 3084-3106).

**Table 9: Identification by means of peptide fingerprint of the most significant mannoproteins of C. albicans between 45 and 66 kDa. The Mw and pI refer to the experimental molecular weight and isoelectric point, respectively.**

| Name | ID | N° | MW | pI | SCORE | Coinciding pep/total pep |
|---|---|---|---|---|---|---|
| Disulfide isomerase | gij68477899 | 1 | 49008 | 4.48 | 121 | 9/12 |
| | | 2 | 46651 | 4.52 | 158 | 13/20 |
| | | 4 | 64149 | 4.78 | 337 | 29/36 |
| | | 5 | 63665 | 4.95 | 361 | 30/34 |
| | | 6 | 60488 | 4.94 | 305 | 23/32 |
| Zinc protein (Zinc Ring finger protein) | gij68478509 | 3 | 57360 | 4.67 | 68 | 5/10 |
| Aminopeptidase Y(APE3) | gij68483101 | 7 | 69731 | 5.12 | 231 | 18/26 |
| | | 8 | 68681 | 5.22 | 280 | 22/32 |
| | | 9 | 68551 | 5.32 | 165 | 14/23 |
| Mitochondrial ketol acid-reductoisomerase | gij68484035 | 10 | 51680 | 5.13 | 312 | 25/31 |
| | | 11 | 49194 | 5.18 | 155 | 13/18 |
| | | 32 | 51290 | 5.26 | 178 | 16/21 |
| Tu elongation factor + Tu translation elongation factor | gij66047777+ gij68473812 | 12 | 57797 | 5.33 | 287 (150 + 98) | 21/22 |
| Action | gij68467327 | 13 | 57797 | 5.45 | 155 | 12/19 |
| Enolase | gij68488457 | 14 | 58458 | 5.55 | 217 | 16/24 |
| | | 15 | 56927 | 5.68 | 299 | 19/21 |
| | | 16 | 53677 | 5.45 | 103 | 7/10 |
| | | 17 | 49288 | 5.57 | 118 | 9/13 |
| | | 21 | 44534 | 5.75 | 120 | 9/15 |
| | | 23 | 46210 | 5.95 | 176 | 12/16 |
| | | 26 | 56176 | 5.96 | 184 | 13/18 |
| | | 33 | 49757 | 5.31 | 150 | 10/13 |
| Alcohol dehydrogenase (ADH1) | gjj68467815 | 18 | 52073 | 5.62 | 177 | 14/25 |
| | | 20 | 51387 | 5.81 | 120 | 9/15 |
| | gij68468132 | 22 | 45343 | 5.86 | 77 | 4/4 |
| 6-phosphogluconate dehydrogenase(COR14) | gij68467359 | 19 | 64393 | 5.99 | 147 | 12/14 |
| Fructose bisphosphate aldolase | gij68466123 | 24 | 47634 | 6.05 | 168 | 11/15 |
| | | 25 | 45257 | 6.15 | 306 | 25/34 |
| 3-phosphoglycerate kinase | gij68489602 | 27 | 57907 | 6.07 | 152 | 14/20 |
| | | 31 | 57469 | 6.26 | 249 | 21/28 |
| Isocytrate dehydrogenase | gij68481517 | 28 | 51095 | 5.96 | 231 | 17/22 |
| Alcohol dehydrogenase 1 + GDP-mannose pyrophosphorylase | gij1168348+ gij68490504 | 29 | 52470 | 6.06 | 278(160+ 97) | 22/28 |
| Glutathione dependent formaldehyde dehydrogenase | gij68473113 | 30 | 56604 | 6.16 | 83 | 6/7 |
| Ubiquinol cyt-c reductase(core portein2) | gij68488175 | 34 | 47996 | 5.35 | 162 | 11/15 |

### 4. Obtaining protein subfractions of different isoelectric point

To enable further specifying in the search of the main mannoproteins responsible for inducing the increase of the tumor adhesion to the endothelium, the crude extract of *C. albicans* was separated into four subfractions differentiated according to the isoelectric point, and the mannoproteins were purified from each of them by means of affinity chromatography in Concanavalin A-Sepharose columns as has been described above. Rotofor system which allows performing preparative isoelectric focusing was used to separate the fractions according to their isoelectric point. The purification of the mannoprotein fractions according to their isoelectric point by means of using the Rotofor and affinity chromatography is the best system for a preparative separation according to the pI of complex protein mixtures, but still there is a considerable loss of the amount of sample therefore the certain percentage of fraction recovery with respect to the starting sample were not very high.

**Table 10: Result from the subfractions obtained after separating the crude extract of C. albicans according to their isoelectric point by means of the Rotofor and after purifying these fractions by means of affinity chromatography with Con-A.**

| | Protein (mg) | MN (mg) | | Protein (mg) | MN (mg) |
|---|---|---|---|---|---|
| | ROTOFOR | | | COLUMN | |
| Starting ext. | 100 | 32 | | | |
| F1 | 4.60 | 5.49 | MNF1 | 0.93 | 3.45 |
| F2 | 3.50 | 1.85 | MNF2 | 0.72 | 0.78 |
| F3 | 4.80 | 1.47 | MNF3 | 0.47 | 0.35 |
| F4 | 11.80 | 1.41 | MNF4 | 0.75 | 0.31 |
| Total | 24.70 | 10.21 | | 2.87 | 4.89 |

For the separation based on the isoelectric point ampholines from a pH range of 3 to 5.6 were used, leaving in the last fraction all the mannoproteins of higher isoelectric point, which has already been observed as not very abundant. Finally, the ranges of pI of each of the fractions were F1: <4.25, F2: 4.25 - 5, F3: 5 -5.6, F4: >5.6.

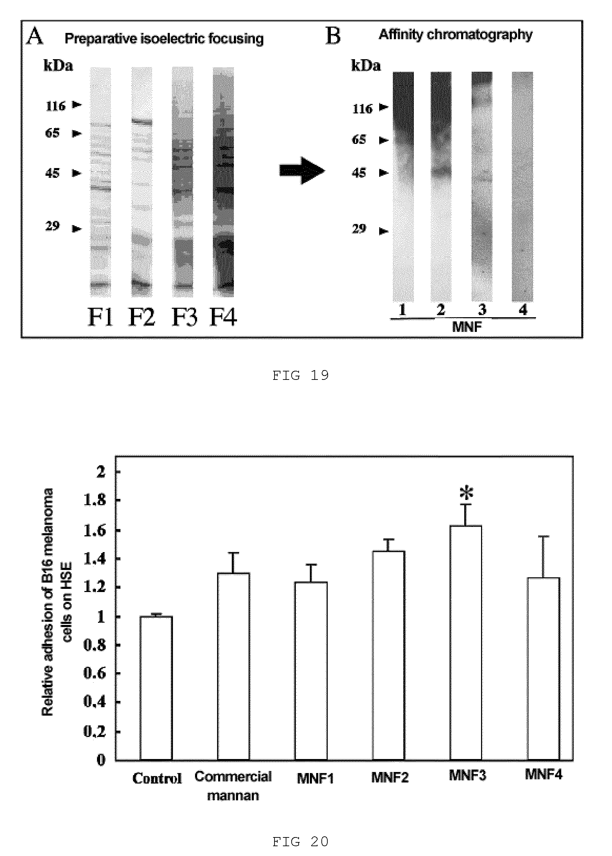

Figure 19 shows the result from the separation of the crude extract into the 4 subfractions according to their isoelectric point and the result after their subsequent purification by means of affinity chromatography in Concanavalin A-Sepharose columns. The separation was satisfactory and, as can be seen in the western blot evaluated against Con A-HRP, the most mannosylated mannoproteins of more than 45 kDa are in acidic isoelectric points, whereas as the isoelectric point increases the greater the reaction is limited to a band of 45 kDa. These results coincide with those obtained in the analysis made by means of two-dimensional electrophoresis of the crude extract, PF and MNF of *C. albicans* of Example 5.

### 5. Effect of the mannoprotein subfractions of C. albicans in the tumor adhesion to the HSE

After obtaining the 4 subfractions of different isoelectric points, their effect in the tumor adhesion to the endothelium was analyzed. To that end, similarly to that performed with the fractions separated according to their molecular weight, the concentration of the fractions was adjusted to the same concentration of carbohydrates to prevent that the difference between the effect of the different fractions was due to the amount of carbohydrates present in each fraction.

According to the data shown in Figure 20, the mannoproteins having the greatest importance in the tumor adhesion are between the mannoproteins identified as part of the subfraction three (MNF3) with an approximate isoelectric point between 5 and 5.6. Therefore, taking into account both the results from the fractioning according to the molecular weight and those obtained from the fractioning according to the isoelectric point, from the total of identified mannoproteins weighing between 45 and 66 kDa, those that exert greatest effect in the tumor adhesion are those that were further identified within of the range of isoelectric point from 5 to 5.6. This reduces the starting list of 15 mannoproteins to a total of 7, from which 4 have been described previously as antigens of *C. albicans.*

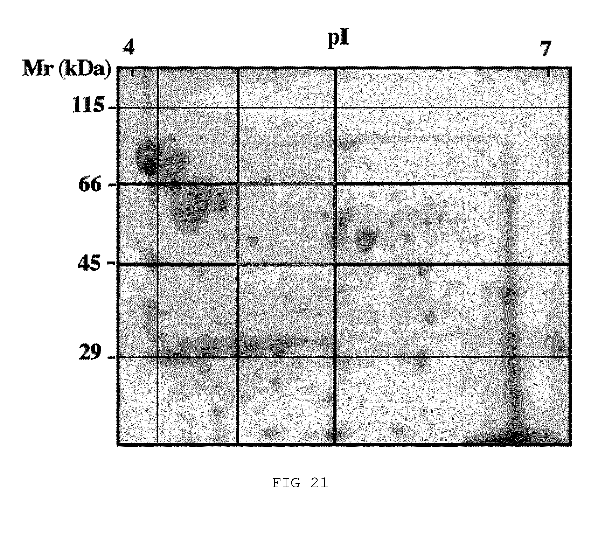

Figure 21 shows a visual reproduction of the fractioning of the mannoproteins of *C. albicans* made according to the molecular weight and the isoelectric point

**Table 11: The Mw and pI refer to the experimental molecular weight and isoelectric point, respectively. The mannoproteins described as antigens are in bold print.**

| NAME | MW(Da) (theoretical) | MW(Da) | pl(theoretical) | pl |
|---|---|---|---|---|
| Aminopeptidase Y | 61100 | 69731 | 4.91 | 5.12 |
| | | 68681 | | 5.22 |
| | | 688551 | | 5.32 |
| Mitochondrial ketol acid-reductoisomerase | 44935 | 51680 | 6.17 | 5,13 |
| | | 49194 | | 5.18 |
| | | 51290 | | 5.26 |
| Tu elongation factor + Tu translation elongation factor | 43701 + 46904 | 57797 | 5.34/5.67 | 5.33 |
| Action | 40382 | 57797 | 5.84 | 5.45 |
| Enolase | 47202 | 58458 | 5.54 | 5.55 |
| Ubiquinol cyt-c reductase (core protein 2) | 39590 | 47996 | 5.45 | 5.35 |
| Alcohol dehydrogenase | 46629 | 52073 | 8.44 | 5.62 |

The mannoproteins which have been described previously in the literature as antigens of *C. albicans* appear in Table 11 labeled in bold print. This means that four proteins from the seven forming this group are antigens. Thus this group of mannoproteins seems to have a high immunogenic potential and may have high endothelial activation. The alcohol dehydrogenase and aminopeptidase were included in the table due to the importance and their proximity to the range of isoelectric point and molecular weight, respectively. The four mannoproteins which have been described as antigens recognized by patient serums are: mitochondrial ketol acid-reductoisomerase, enolase, ubiquinol cyt-c reductase and alcohol dehydrogenase. The 3 mannoproteins of the group which still have not been described as antigens are the aminopeptidase, which is found between the MNF3 and MNF4, the actin, which is a protein forming an essential part of the cell cytoskeleton, and the Tu elongation factor, belonging to a family of proteins which is related with antigenicity, in fact, some elongation factors have already been described as antigens of *C. albicans.*

Conclusions: The mannoprotein fractions of *C. albicans* comprised between 45 and 66 kDa according to the molecular weight and between 5 and 5.6 according to the isoelectric point are those that induce the most increase of the tumor adhesion to the hepatic sinusoidal endothelium. In that region 7 mannoproteins were identified, 4 of them with antigenic potential.

### Example 7

### Comparative two-dimensional analysis of the mannoprotein fractions MNF3 from the different strains

A comparative analysis of the antigen expression of the MNF3 among the strains UPV1413, UPV1360 and NCPF3153 of *C. albicans* was performed. The strain UPV1413 was chosen for the analysis due to its high capacity for stimulating the tumor adhesion to the endothelium, whereas the reference strain NCPF3153 was selected for having the opposite characteristic, since it had not shown the capacity of increasing the adhesion of tumor cells to the HSE. The strain UPV1360 showed capacity of increasing the tumor adhesion, although to a lesser extent than the UPV1413. To perform the analysis at least 3 gels having isoelectric point comprised between 4 and 7 were used for each strain and they were analyzed by means of the Image Master 2D Platinum computer system (GE Healthcare).

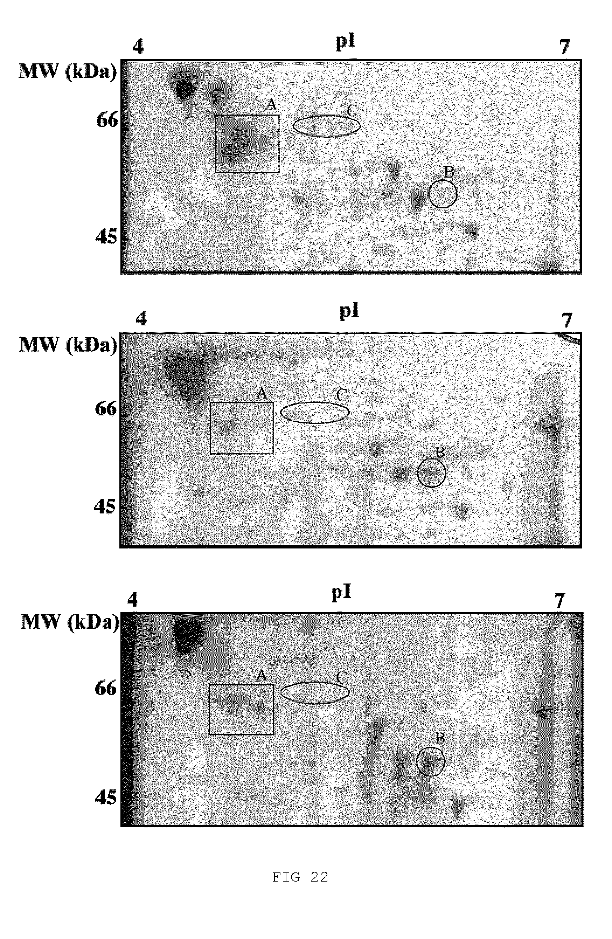

As can be observed in Figure 22, the results showed few significant differences in the expression of the antigen analyzed. The biggest difference of expression was found in the aminopeptidase, which was expressed as one of the most significant proteins of the MNF3 of the strain UPV1413 and it could hardly be distinguished in the MNF3 from the strain NCPF3153. The aminopeptidase has not been described in the literature as antigen of *C. albicans.* Its molecular weight was observed between 68.5 and 69.7 kDa and its isoelectric point between 5.12 and 5.32, placing it within the fraction of isoelectric point which had shown great capacity for increasing the tumor adhesion to the endothelial cells, but is in the upper limit of the MNF3 in terms of the fractions separated according to their molecular weight. When the expression of this mannoprotein in the strain UPV1360 was analyzed, the tumor adhesion did increase instead of being expressed as it was in the strain UPV1413, the expression thereof reduced significantly. Something similar happened with the protein disulfide isomerase, the expression of which was reduced in the NCPF3153 with respect to the UPV1413, and its expression was also reduced in the UPV1360 although the expression was maintained above the level of the reference strain.

The contrary case is found in one of the subunits of the enolase. The strain NCPF3153 and, above all, the strain UPV1360 expressed in large proportion one of the subunits of the enolase, specifically the most basic of them which had not been identified in the strain UPV1413. Therefore, differences of expression have been found in three mannoproteins of the MNF3 such as the enolase, the protein disulfide isomerase and the aminopeptidase, although it cannot be clearly related with a great increase of the tumor adhesion to the HSE. However, the fact that two of these three proteins are expressed both in the strain UPV1413 and in the strain UPV1360, in the first the protein disulfide isomerase (A) and the aminopeptidase (C), and in the second the protein disulfide isomerase (A) and the basic subunit of the enolase (B), in a greater extent as in the reference strain and in all the studies cases, allows assuming an effect thereof in increasing the tumor adhesion.

Conclusion: The two-dimensional analysis of the MNF3 from the different strains provides significant differences of expression between strains of *C. albicans* inducing an effect in increasing the tumor adhesion to the endothelium of the strains that do not induce it.

### Example 8

### Study of the effect of the mannoprotein fraction of C.albicans in tumor proliferation

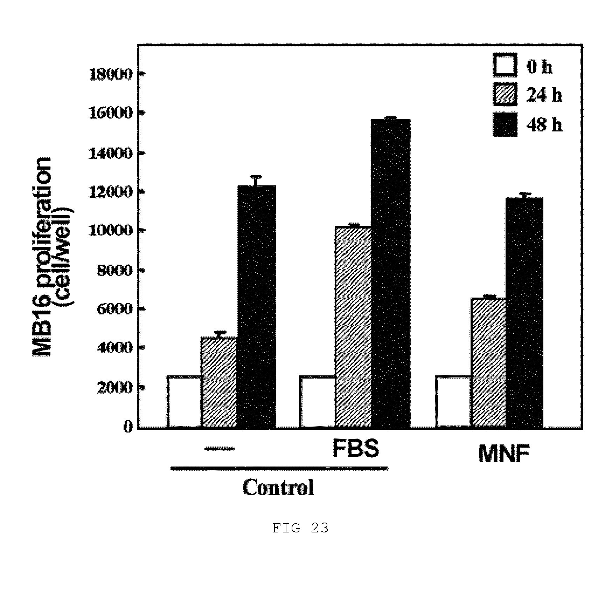

In the present invention the effect of the mannoproteins of *C. albicans* in increasing the tumor adhesion to the hapatic endothelium has been demonstrated. To eliminate a possible direct effect of the mannoproteins of *C. albicans* on the B16 tumor cells and on the endothelium, whether it is possible to induce any variation in the proliferation of the tumor cells was measured. To that end, the MB16 cell proliferation induced by the mannoprotein fraction of *C. albicans* was measured at 24 and 48 hours. The results obtained are shown in Figure 23. It can be seen therein that the MB16 cells incubated with the mannoprotein fraction did not have a proliferation rate higher than the control cells. Despite that a proliferation greater than the control was produced at 24 hours, this was not corroborated at 48 hours. In both cases, the proliferation induced by the mannoprotein fraction was lower than that induced by the fetal bovine serum.

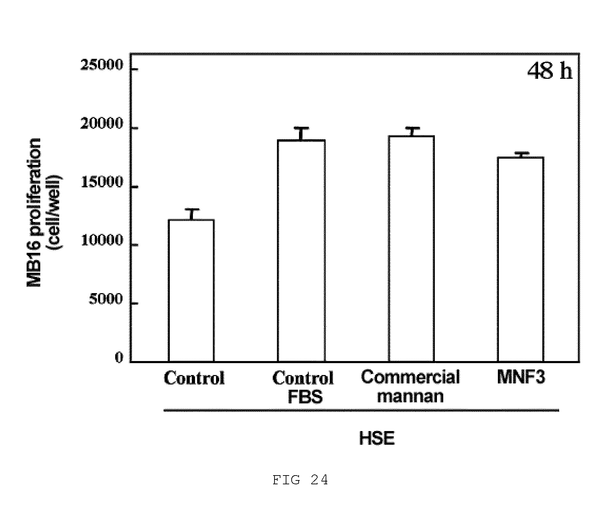

These results were corroborated after performing the test using the MNF3 (45-66 kDa) to stimulate the proliferation. Although in this case, the results shown in Figure 24 indicate that the MNF3 increases the proliferation with respect to the control situation, they also demonstrate that the MNF3 of *C. albicans* do not have capacity for inducing the proliferation of the tumor cells as much as the medium supplemented with FBS or the commercial mannan.

Conclusion: The mannoprotein fraction of *C. albicans* does not induce a significant increase of the proliferation of the MB16 tumor cells.

### Example 9

### Influence of the IL-1β receptor in the tumor adhesion and production of TNF-α induced by the MNF

IL-1β is a proinflammatory molecule which is related with the TNF-α because they both participate in the proinflammatory response of the endothelium and, according to the literature, in the proinflammatory response activated through the mannose receptor, the synthesis and release of TNF-α is prior to that of IL-1β (Vidal-Vanaclocha et *al.,* 2000 cited at *supra*).

The present experiment was conducted with an IL-1β receptor antagonist molecule called IL-1Ra (IL-1 *Receptor antagonist*) which has already been used for such purpose (Vidal-Vanaclocha et *al.* 2000). By means of this molecule the IL-1β receptor was blocked and the repercussion that this has in increasing the tumor adhesion and the production of TNF-α released by the endothelium when it is incubated with the mannoproteins of *C. albicans* was measured.

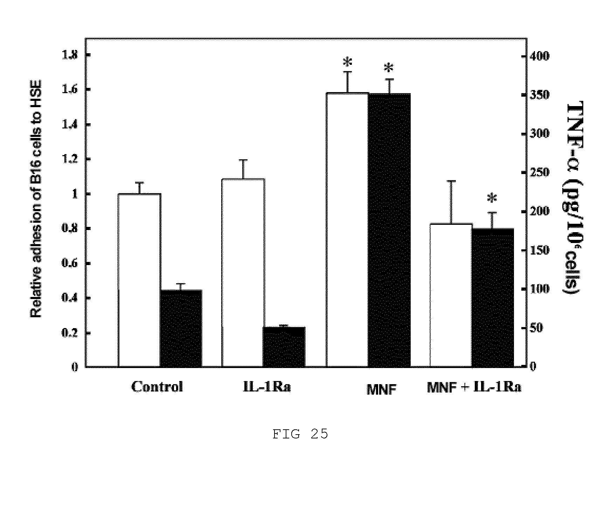

Figure 25 demonstrates that the blocking of the IL-1β receptor completely inhibited the effect that the mannoproteins have on the tumor adhesion. While the adhesion of tumor cells increased to 60% when the endothelium is activated with the MNF, this effect was completely inhibited as the function of the IL-1β receptor is neutralized. This means that the mannoproteins of *C. albicans* trigger the cascade either at that point or before that receptor. It is however surprising that the production of TNF-α also reduced significantly when the IL-1R is blocked. The expected release of TNF-α which will not be affected if the actuation route of the cytokines was exclusively the mannose receptor was also reduced. In this case contrarily to what happened with the tumor adhesion, the reduction was not complete, and maintained, despite the reduction, a production of TNF- α significantly greater than the control situation.

In view of the results obtained, the possibility of the existence among these purified mannoproteins of *C. albicans* of a mannoprotein which could be bound to this receptor and directly activating it was proposed. To that end, for attempting to identify a mannoprotein related with the activation of the IL-1β receptor, the anti-mouse IL-1β monoclonal antibody (R&D systems) which reacts against the mouse IL-1β by neutralizing its biological function and therefore, preventing the same from binding to its receptor was used. Starting from the idea that in order to specifically neutralize the biological function of the IL-1β the antibody could recognize the epitope used by the cytokine for binding with its receptor, this antibody was also used to recognize, if any, a mannoprotein of *C. albicans* capable of binding and activating the IL-1R. With this objective a two-dimensional Western blot of the mannoproteins of *Candida* in comparison to the anti-mouse IL-1β antibody was performed.

The anti IL-1β antibody recognized a mannoprotein which by molecular weight coincided with the fraction MNF3 described in this invention as having the greatest capacity for stimulating the HSE and inducing an increase of the tumor adhesion to this endothelium. Furthermore, the molecular weight thereof is located approximately in the area which has served as limit between the MNF3 and MNF2, which also showed a significant effect. By paying attention to the isoelectric point thereof, the mannoprotein was also detected within the range of the fraction showing greater effect. Therefore, this mannoprotein was an important candidate to be the endothelium stimulator and therefore, stimulator of the increase of the tumor adhesion to the same.

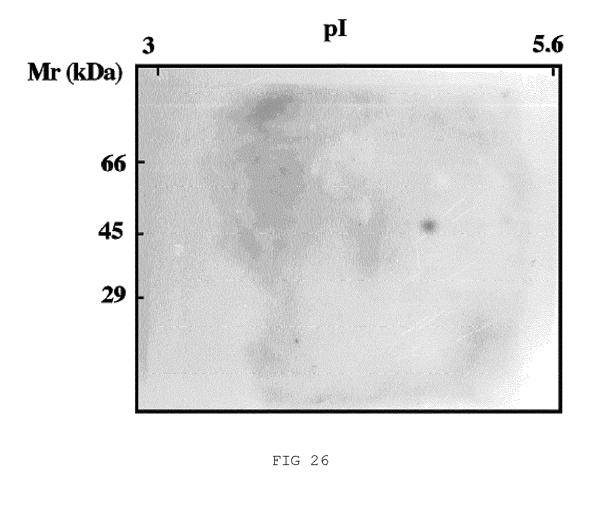

The reaction obtained was reproducible in all the Western blot performed. However, said reaction was not clearly visible in the gels stained with Coomassie blue (Figure 26). Despite that, the fragment of gel which provided reaction was manually extracted and sequenced by means of LC-MS/MS in the proteomic service of the UPV/EHU.

Conclusion: The IL-1β receptor antagonist (IL-Ra) completely inhibits the increase of the tumor adhesion to the hepatic sinusoidal endothelium induced by the MNF. Furthermore, it partially reduces the release of TNF-α by the endothelium stimulated by the mannoproteins of *C. albicans.*

## Claims

1. Use of an antifungal agent in the preparation of a pharmaceutical composition for treating a disease associated to an unwanted cell adhesion.

2. Use according to claim 1, wherein the disease associated to an unwanted cell adhesion is a tumor process, a metastatic process, an inflammatory process preferably mediated by cytokines, restenosis or a disease causing a cellular immune response.

3. Use according to claim 2, wherein the metastatic process is originated from liver tumor.

4. Use according to any of claims 1 to 3, wherein the antifungal agent is selected from a polyene, an azole, an allylamine, a lipopeptide and a fluorinated pyrimidine.

5. Use of a compound capable of binding to a mannoprotein or to an equivalent functional variant thereof, and blocking the binding of said mannoprotein or an equivalent functional variant thereof, to an endothelial cell receptor in the preparation of a pharmaceutical composition for preventing and/or treating a disease associated to an unwanted cell adhesion.

6. Use according to claim 5, wherein the mannoprotein comes from the mannoprotein fraction of a yeast, preferably, of a yeast from the genus Candida, more preferably from *Candida albicans.*

7. Use according to claim 6, wherein the mannoprotein fraction comprises at least a mannoprotein having a molecular weight between 30 and 45 kDa or between 45 and 66 kDa.

8. Use according to claim 7, wherein the mannoprotein having a molecular weight between 45 and 66 kDa of molecular weight is a mannoprotein selected from disulfide isomerase, zinc protein, aminopeptidase Y, mitochondrial ketol acid-reductoisomerase, Tu elongation factor, Tu translation elongation factor, actin, enolase, alcohol dehydrogenase, 6-phosphogluconate dehydrogenase, fructose bisphosphate aldolase, 3-phosphoglycerate kinase, isocytrate dehydrogenase, GDP-mannose pyrophosphorylase, glutathione dependent formaldehyde dehydrogenase and ubiquinol cyt-c reductase .

9. Use according to claim 6, wherein the mannoprotein fraction comprises at least a mannoprotein having a molecular weight between 45 and 66 kDa, and a point between 5 and 5.6.

10. Use according to claim 9, wherein the mannoprotein subfraction comprises at least a mannoprotein selected from aminopeptidase Y, mitochondrial ketol acid-reductoisomerase, Tu elongation factor, Tu translation elongation factor, actin, enolase, ubiquinol cyt-c reductase and alcohol dehydrogenase.

11. Use according to any of claims 5 to 10, wherein the endothelial cell receptor is a proinflammatory cytokine receptor preferably the interleukin 1-beta receptor (IL1-β).

12. Use according to any of claims 5 to 11, wherein the disease associated to an unwanted cell adhesion is a tumor process, a metastatic process, an inflammatory process preferably mediated by cytokines, restenosis or a disease causing a cellular immune response.

13. Use according to claim 12, wherein the metastatic process is originated from liver tumor.

14. Use according to any of claims 5 to 13, wherein the compound capable of binding to a mannoprotein or an equivalent functional variant thereof, and blocking its binding to a proinflammatory cytokine receptor is an antibody.

15. Use according to any of claims 5 to 14, wherein the pharmaceutical composition comprises one or more pharmaceutically acceptable excipients.

16. Use according to any of claims 5 to 15, wherein the pharmaceutical composition further comprises an antifungal compound.

17. Method for identifying a compound potentially useful for preventing and/or treating a disease associated to an unwanted cell adhesion, which comprises contacting an endothelial cell receptor and a mannoprotein or an equivalent functional variant thereof, and selecting a compound blocking the binding of said mannoprotein or equivalent functional variant thereof to said endothelial cell receptor.

18. Method according to claim 17, wherein the mannoprotein comes from the mannoprotein fraction of a yeast, preferably of a yeast from the genus Candida, more preferably from *Candida albicans.*

19. Method according to claim 18, wherein the mannoprotein fraction comprises at least a mannoprotein having a molecular weight between 30 and 45 kDa or between 45 and 66 kDa.

20. Method according to claim 19, wherein the mannoprotein having a molecular weight between 45 and 66 kDa of molecular weight is a mannoprotein selected from disulfide isomerase, zinc protein, aminopeptidase Y, mitochondrial ketol acid-reductoisomerase, Tu elongation factor, Tu translation elongation factor, actin, enolase, alcohol dehydrogenase, 6-phosphogluconate dehydrogenase, fructose bisphosphate aldolase, 3-phosphoglycerate kinase, isocytrate dehydrogenase, GDP-mannose pyrophosphorylase, glutathione dependent formaldehyde dehydrogenase and ubiquinol cyt-c reductase.

21. The method according to claim 18, wherein the mannoprotein fraction comprises at least one mannoprotein having a molecular weight between 45 and 66 kDa and a point between 5 and 5.6.

22. Method according to claim 21, wherein the mannoprotein having a molecular weight between 45 and 66 kDa and a point between 5 and 5.6 comprises, at least a mannoprotein selected from aminopeptidase Y, mitochondrial ketol acid-reductoisomerase, Tu elongation factor, Tu translation elongation factor, actin, enolase, ubiquinol cyt-c reductase and alcohol dehydrogenase.

23. Method according to any of claims 17 to 22, wherein the endothelial cell receptor is a proinflammatory cytokine receptor, preferably, the interleukin 1-beta receptor (IL1-β).

24. Method according to any of claims 17 to 23, wherein the disease associated to an unwanted cell adhesion is a tumor process, a metastatic process, an inflammatory process preferably mediated by cytokines, restenosis or a disease causing a cellular immune response.

25. Method according to claim 24, wherein the metastatic process is originated from liver tumor.

26. Method according to any of claims 17 to 25, wherein the compound capable of binding to a mannoprotein or an equivalent functional variant thereof, and blocking its binding to a proinflammatory cytokine receptor is an antibody.

27. Use of kit comprising a compound capable of binding to a mannoprotein or to an equivalent functional variant thereof, and blocking the binding of said mannoprotein or equivalent functional variant thereof, to an endothelial cell receptor for preventing and/or treating a disease associated to an unwanted cell adhesion.

28. Use according to claim 27, wherein the mannoprotein comes from the mannoprotein fraction of a yeast, preferably of a yeast from the genus Candida, more preferably from *Candida albicans.*

29. Use according to claim 28, wherein the mannoprotein fraction comprises at least a mannoprotein having a molecular weight between 30 and 45 kDa or between 45 and 66 kDa.

30. Use according to claim 29, wherein the mannoprotein having a molecular weight between 45 and 66 kDa is a mannoprotein selected from disulfide isomerase, zinc protein, aminopeptidase Y, mitochondrial ketol acid-reductoisomerase, Tu elongation factor, Tu translation elongation factor, actin, enolase, alcohol dehydrogenase, 6-phosphogluconate dehydrogenase, fructose bisphosphate aldolase, 3-phosphoglycerate kinase, isocytrate dehydrogenase, GDP-mannose pyrophosphorylase, glutathione dependent formaldehyde dehydrogenase and ubiquinol cyt-c reductase.

31. Use according to claim 28, wherein the mannoprotein fraction comprises at least a mannoprotein having a molecular weight between 45 and 66 kDa and an isoelectric point between 5 and 5.6.

32. Use according to claim 31, wherein the mannoprotein having a molecular weight between 45 and 66 kDa and an isoelectric point between 5 and 5.6 comprises, at least a mannoprotein selected from aminopeptidase Y, mitochondrial ketol acid-reductoisomerase, Tu elongation factor, Tu translation elongation factor, actin, enolase, ubiquinol cyt-c reductase and alcohol dehydrogenase.

33. Use according to any of claims 27 to 32, wherein the endothelial cell receptor is a proinflammatory cytokine receptor, preferably, the interleukin 1-beta receptor (IL1-β).

34. Use according to any of claims 27 to 33, wherein the disease associated to an unwanted cell adhesion is a tumor process, a metastatic process, an inflammatory process preferably mediated by cytokines, restenosis or a disease causing a cellular immune response.

35. Use according to claim 34, wherein the metastatic process is originated from liver tumor.

36. Use according to any of claims 27 to 35, wherein the compound capable of binding to a mannoprotein, or an equivalent functional variant thereof, and blocking its binding to a proinflammatory cytokine receptor is an antibody.

37. In vitro method for predicting the probability of a patient suffering from cancer to suffer from metastasis, which comprises detecting a yeast or a mannoprotein, or an equivalent functional variant thereof, in a sample from said patient, wherein if said yeast or mannoprotein, or equivalent functional variant thereof is detected, then said patient has a high probability of suffering metastasis.

38. In vitro method for designing a personalized therapy for a patient suffering from cancer which comprises detecting a yeast or a mannoprotein or an equivalent functional variant thereof in a sample from said patient, wherein if said yeast or mannoprotein or functional variant thereof is detected, then the patient is a candidate for receiving a treatment based on an antifungal agent or on a compound capable of binding to a mannoprotein, or to an equivalent functional variant thereof, and blocking the binding of said mannoprotein or equivalent functional variant thereof to an endothelial cell receptor.
